# FASCICULE DE BREVET EUROPEEN

(11) **EP 1 644 731 B1**
(45) Date de publication et mention de la délivrance du brevet: **20.08.2008**
(21) Numéro de dépôt: 04767642.4
(22) Date de dépôt: 09.07.2004
(51) Int. Cl.: G01N 33/50, G01N 33/543, G01N 33/68

(54) **PROCEDE ET DISPOSITIF POUR L ANALYSE DE MILIEUX REACTIONNELS VIVANTS**
VERFAHREN UND VORRICHTUNG ZUR ANALYSE LEBENDER REAKTIONSMEDIEN
METHOD AND DEVICE FOR THE ANALYSIS OF LIVING REACTION MEDIA

(30) Priorité: 11.07.2003 FR 0308526
(43) Date de publication de la demande: 12.04.2006
(73) Titulaire: COMMISSARIAT A L'ENERGIE ATOMIQUE, 75015 Paris (FR)
(72) Inventeur: REBOUD, Julien, Singapore 128035 (SG); SCHAACK, Béatrice, F-38000 Grenoble (FR); CHATELAIN, François, 38470 Beaulieu (FR)
(74) Mandataire: Corizzi, Valérie
(86) Numéro de dépôt international: PCT/FR2004/001810
(87) Numéro de publication internationale: WO 2005/008238

(56) Documents cités:
- EP-A- 1 284 495
- WO-A-01/26460
- WO-A-01/84143
- WO-A1-99/55827
- US-A1- 2002 158 196

## Description

La présente invention a pour objet un procédé et un dispositif pour l'analyse de milieux réactionnels comportant une ou plusieurs cellules, ce procédé et ce dispositif permettant de réaliser une analyse automatisée à haut débit.

Actuellement dans la recherche en biologie, et notamment dans le domaine pharmaceutique, on cherche à analyser le phénotype de populations cellulaires, et plus particulièrement le protéome, en réponse à une ou plusieurs stimulations exercées sur ces populations cellulaires, de façon à évaluer l'impact de ces stimulations sur les cellules auxquelles elles sont appliquées. Cette analyse requiert des outils permettant la mise en oeuvre de réactions sur des milieux réactionnels vivants (les populations de cellules) et l'analyse de ces milieux réactionnels dans des conditions telles qu'il n'y ait pas (ou le moins possible) de déformation du milieu réactionnel entre ces deux étapes, de façon à limiter la perte ou la transformation de l'information.

Il existe dans ce domaine de la recherche une exigence croissante en termes de débit de traitement des échantillons. Des molécules de plus en plus nombreuses sont disponibles pour être testées, des systèmes cellulaires de plus en plus variés (tissus, réseaux, cellules) sont disponibles pour être étudiés.

Le besoin existe donc pour des outils d'analyse de milieux réactionnels vivants, qui permettent une étude de ces milieux réactionnels dotée de la plus grande objectivité possible et d'une vitesse de traitement aussi élevée que possible.

La présente invention qui allie la culture de populations de cellules sur des supports matriciels et l'analyse par spectrométrie de masse permet de répondre à cette attente.

A l'heure actuelle, le criblage phénotypique se fait essentiellement en utilisant des méthodes colorimétriques, des molécules fluorescentes ou radioactives. Ces méthodes nécessitent le marquage de molécules spécifiques, qui impose de connaître a priori la (ou les) protéine(s) recherchée(s). La présente invention ne nécessite pas de connaissances préalables sur les modifications attendues des cellules suite aux stimuli.

De nombreuses méthodes permettent aujourd'hui d'analyser plus ou moins directement le phénotype d'une ou plusieurs cellules avec un débit plus ou moins rapide.

Les analyses utilisant directement des cellules ont toutes en commun un principe de fonctionnement, mais diffèrent sur le signal analysé en sortie, qui représente le phénotype :
- signal optique : fluorescence, luminescence, colorimétrie.
- Signal radioactif : molécules marquées.
- Signal électrique : électrophysiologie.

Ces différentes méthodes d'analyse sont généralement implémentées dans un format puits de plastique (96 ou 384, puits par plaque) qui nécessite encore beaucoup de réactifs (0,1 à 0,5ml par puit dans une plaque 96) et ne permet qu'un débit modéré.

De plus le signal n'est pas directement représentatif du phénotype, mais nécessite un étalonnage souvent complexe.

Il est par ailleurs difficile d'analyser directement les sécrétions des cellules, sauf par des méthodes de capture par anticorps.

L'analyse des différents phénotypes ne se fait que sur la base d'un seul paramètre, une molécule que l'on connaît, dont on veut s'assurer de la présence ou de l'absence : on est obligé au départ de savoir quelle molécule est recherchée. Par exemple, lorsque l'on veut étudier la présence d'une molécule dans la cellule, on doit au départ avoir une idée assez précise des différentes propriétés de cette molécule, pour la coupler à un anticorps spécifique ou rendre la molécule fluorescente. On peut aussi rechercher des modifications post-traductionnelles des protéines, mais en ciblant une modification particulière.

La recherche relative à la limite de vitesse de réalisation et de traitement de ces différentes méthodes a conduit au développement d'un format plus apte à un très haut-débit de manipulation. Le premier exemple d'aboutissement de cette recherche se retrouve dans les puces à ADN : les ARN messagers exprimés par une population cellulaire (après stimulation) sont criblés sous forme de dépôts sur un support matriciel, ou puce, contenant des fragments d'ADN potentiellement complémentaires de cette expression. Cette méthode permet d'apprécier le niveau d'expression de plusieurs milliers de gènes sur une puce.

Cependant les résultats ne sont souvent pas assez démonstrateurs pour pouvoir se passer d'une étude plus fine après une première analyse. En effet, cette méthode implique des étapes de traitements des échantillons (recueil et multiplication de la quantité d'ARN, PCR inverse) qui éloignent du modèle cellulaire considéré. En outre elle ne permet d'analyser que le niveau d'expression d'ARN, qui n'est pas directement en rapport avec la quantité de protéines produites, ni avec leurs qualités, à cause notamment de modifications post-traductionnelles (épissage alternatif, conformation 3D quaternaire modifiée, phosphorylation, assemblages).

D'autres puces à molécules tentent de palier cet éloignement, en essayant d'analyser directement le niveau d'expression de différentes molécules au sein de la culture cellulaire. Ainsi diverses molécules ont été déposées de façon matricielle sur des supports sous le format puce : ARN, protéines, sucres par exemple. Malheureusement les techniques mises en oeuvre ne sont pas véritablement fiables, et sont destinées à analyser des interactions entre molécules plutôt qu'un phénotype cellulaire.

Enfin, même si le nombre de molécules différentes analysées sur une puce est grand, un choix a déjà été fait quant aux molécules que l'on dépose sur le support. Cette démarche implique une connaissance a priori des phénotypes recherchés, qui restreint les possibilités d'analyses.

En ce qui concerne les puces à cellules, elles ont été décrites par Sabatini *et al.* Ces puces à cellules fonctionnent de la façon suivante : on dépose de l'ADN sous forme d'une dispersion dans de la gélatine de façon matricielle sur une lame de verre. Après séchage, les emplacements comprenant l'ADN sont traités par un agent de transfection lipidique puis la plaque est placée dans un milieu dans lequel sont réparties des cellules. Sur la lame de verre, l'ADN gélatinisé est présent sous forme solide et la transfection se fait en phase semi-solide en liant les molécules adjacentes aux dépôts d'ADN à des lipides favorisant la pénétration de l'ADN dans les cellules adjacentes aux dépôts d'ADN. On obtient une matrice de cellules transfectées aux emplacements correspondant aux dépôts d'ADN. Cette méthode présente l'inconvénient d'être peu précise et non reproductible. L'accrochage par la gélatine ne permet pas de contrôler le décrochage de l'ADN transfecté. Il ne permet pas non plus d'améliorer l'efficacité de la transfection. Par ce procédé l'expression ou le blocage de l'expression d'une quantité suffisante de protéine peut difficilement être obtenu. Une seule sorte de cellule peut être utilisée pour chaque lame de verre. On ne peut pas, par ce moyen, étudier l'interaction entre cellules ou l'interaction entre des cellules et des réactifs autres que de l'ADN.

Par rapport aux méthodes d'analyse énumérées ci-dessus, la spectrométrie de masse constitue un outil beaucoup plus intéressant en terme de relation entre le signal détecté et le phénotype cellulaire (les molécules exprimées par la cellule et leurs quantités respectives). Elle permet en effet de mesurer directement la quantité de protéines présentes dans un échantillon, sans qu'il soit nécessaire de modifier l'intégrité de la molécule (par un marquage de la molécule par exemple). Le principe consiste à désorber d'un support solide et ioniser des molécules de l'échantillon à analyser. Ensuite le rapport masse/charge des particules ainsi créées est recueilli ; il représente la signature de la molécule désorbée.

Dans l'Utilisation qui en est faite en biologie cellulaire et en protéomique actuellement, de nombreux traitements des échantillons sont nécessaires (lyse cellulaire, purification des échantillons, introduction d'une matrice pour la spectrométrie MALDI par exemple) car la spécificité et la précision que permet pour l'instant l'instrumentation n'autorisent pas une analyse directe des échantillons, dont la qualité n'est pas assez contrôlée. Ces traitements introduisent des biais dans l'analyse, qui n'est plus vraiment représentative du phénotype cellulaire.

De plus l'implémentation de ces méthodes et l'utilisation de l'appareillage existant ne permettent qu'un très faible débit de traitement des échantillons.

Un nouveau système (le **SELDI** pour « surface enhanced laser desorption ionisation ») permettant d'utiliser la spectrométrie de masse sur des échantillons plus ou moins complexes se rapprochant un peu plus du modèle cellulaire a été développé récemment. L'atout de ce système vient de sa capacité à réaliser une partie des purifications nécessaires à l'obtention d'une analyse correcte directement sur le support qui rentre dans la machine (adsorption sélective et orientée en surface du support), ce qui permet d'écourter et de simplifier les traitements d'échantillons.

Ce système est utilisé notamment dans la découverte de marqueur de maladie: des échantillons d'une population malade sont analysés par rapport à d'autres d'une population saine, et les différences sont analysées par un logiciel propriétaire ; cela permet d'identifier des marqueurs de la maladie, et donc des cibles potentiellement intéressantes. Cette méthode permet de faire une analyse vraiment globale des échantillons : on ne connaît pas a priori les molécules qui seront les marqueurs de la maladie.

Cependant, des traitements d'échantillons sont encore nécessaires (lyse cellulaire, lavages et purifications) pour avoir de bons résultats. Par exemple la culture des cellules est réalisée en dehors du substrat, ce qui implique un biais non contrôlé de transfert d'échantillons. Par ailleurs, le débit d'analyse est encore faible : les puces utilisables dans ce système ne peuvent contenir que 16 plots différents à ce jour.

Plusieurs documents concernant la spectrométrie de masse sont relatifs à l'analyse de milieux réactionnels vivants :
- Le document US-2002/0160420 décrit l'analyse par spectrométrie de masse d'un échantillon de sérum humain ayant subi plusieurs étapes de purifications.
- Le document US-2002/0076739 décrit un procédé d'analyse de protéines dans des mélanges. Des réactifs marqués spécifiques de certains peptides sont mis à réagir avec des mélanges de protéines, les molécules ayant réagi sont isolées puis analysées par spectrométrie de masse.
- Le document DE 10038684 décrit un procédé pour identifier des microorganismes à l'aide d'un système MALDI-TOF-MS, dans lequel le spectre d'un échantillon d'un microorganisme à identifier est comparé à une base de données de spectres de référence.
- Le document US-6,531,318 décrit un procédé d'analyse de tissus biologiques, ce procédé comportant une étape de microdissection au moyen d'un laser, cette microdissection permettant de sélectionner des agrégats de cellules, suivie d'une analyse par spectrométrie de masse.
- Le document WO 00/48004 décrit un dispositif pour l'analyse de matériau cellulaire. Des cellules sont mises en culture, purifiées par des méthodes autres que la chromatographie, puis injectées dans un spectromètre de masse.
   Cette méthode requiert la manipulation des échantillons de culture de cellule, notamment la purification est une étape de procédé qui élimine certains constituants de l'échantillon, sans que l'on maîtrise parfaitement la sélection des composants qui sont éliminés.
- Le document WO 02/103360 décrit une méthode d'analyse de protéines à la surface d'une cellule, cette méthode comprend la mise en réaction de la cellule avec une substance à sa surface et son analyse par spectrométrie de masse.
- Le document WO 01/65254 décrit un procédé pour identifier la structure chimique d'une substance présente dans des tissus ou des cellules de différents organismes, ce procédé comprenant l'irradiation d'une zone précise d'une coupe de tissu vivant ou d'une cellule de façon à ioniser la substance et à réaliser son spectre de masse et l'analyse de ce spectre de façon à identifier sa structure.
- Le document WO 02/101356 décrit un procédé d'analyse de protéines mitochondriales. Les protéines constituant la mitochondrie sont séparées par gel d'électrophorèse en 2 dimensions, puis analysées par spectrométrie de masse.
- Le document WO 01/84143 décrit un procédé pour analyser un grand nombre de protéines en un temps réduit. Des cellules sont soumises à une stimulation, lysées et les échantillons divisés de façon à obtenir des lots de quelques centaines de protéines puis ces lots sont analysés par spectrométrie de masse à l'aide d'une batterie de spectromètres en parallèle. l'introduction de la page 5a ici

Les méthodes d'analyse par spectrométrie de masse de milieux réactionnels vivants comportent tous une ou plusieurs étapes de purification et/ou de manipulation qui entraînent une perte d'information et/ou elles supposent la recherche de molécules bien définies tandis que l'un des objectifs de l'invention était l'obtention d'une analyse sans contrainte des données exprimées par le milieu réactionnel vivant.

En outre, ces méthodes de l'art antérieur, en raison des étapes de purification et/ou de manipulation qu'elles comportent, sont peu propices à un traitement à haut débit.

Par rapport aux techniques de l'art antérieur, le procédé et le dispositif de l'invention présentent de nombreux avantages :
- possibilité de travailler sur un assortiment de cellules variées disposées sur une même puce,
- mise en parallèle de systèmes cellulaires variés,
- suppression ou limitation des étapes susceptibles de biaiser l'analyse (telles que purification, lyse de cellules, déplacement d'un échantillon d'un milieu à un autre),
- possibilité d'analyser un très grand nombre d'échantillons en un temps réduit,
- Le document WO 99/55827 décrit un dispositif et un procédé d'analyse de milieux réactionnels comportant au moins une cellule, cette (ou ces) cellule(s) étant déposée(s) sur un support permettant le maintien en vie de dite(s) cellule(s) (sous forme d'une goutte), leur mise en réaction et leur analyse. La goutte de cellule(s) est protégée par un film ("a cover plate") qui empêche l'évaporation mais qui est perméable au gaz.
- obtention de données non déformées par rapport aux informationspar rapport aux informations émises par la cellule.

L'invention a donc pour objet un procédé d'analyse d'au moins un milieu réactionnel comportant au moins une cellule C, ledit procédé étant caractérisé en ce que
on utilise un supports S comportant une surface sensiblement cette surface comportant au moins un moyen destiné à la reception des gouttes aqueuses, (i) la cellule C est déposée
   sous forme d'une goutte aqueuse sur un moyen destiné à la reception des gouttes du supports S à l'aide d'un moyen choisi parmi : de fin capillaires, un systéme prézoéléctrique;
(ii) la surface sensiblement plane du support S sur laquelle a été déposée la goutte aqueuse contenant la cellule C est éventuellement recouverte par un film de séparation F, permettant le passage des gaz et empêchant l'évaporation des gouttes aqueuses déposées sur le support S ;
(iii) la cellule C est éventuellement soumise à une stimulation ;
(iv) le milieu réactionnel est préparé et introduit dans le spectromètre de masse ;
(v) le milieu réactionnel est désorbé et ionisé ;
(vi) le spectre de masse du milieu réactionnel est recueilli et analysé.

L'invention a également pour objet un dispositif permettant l'analyse d'au moins un milieu réactionnel comportant au moins une cellule C, ce dispositif étant caractérisé en ce qu'il comporte :
- un support S comportant une surface sensiblement plane éventuellement recouverte d'un film de séparation F permettant le passage des gaz et empêchant l'évaporation des gouttes aqueuses déposées sur le support S,
- des moyens permettant le dépôt sur ladite surface, et éventuellement sous le film F, de gouttes aqueuses contenant la cellule C,
- des moyens permettant la désorption et l'ionisation du milieu réactionnel,
- un spectromètre de masse.

Le dispositif de l'invention peut en outre éventuellement comprendre une enceinte à atmosphère contrôlée dans laquelle est placée le support S de façon à permettre la survie de la cellule C.

La stimulation à laquelle est soumise la cellule C peut être de différentes natures. Elle peut consister en :
- l'introduction d'un réactif R,
- la mise en contact avec une ou plusieurs cellules,
- un apport d'énergie,
- l'application d'un champ électrique ou d'un champ magnétique,
- une stimulation par traitement optique.

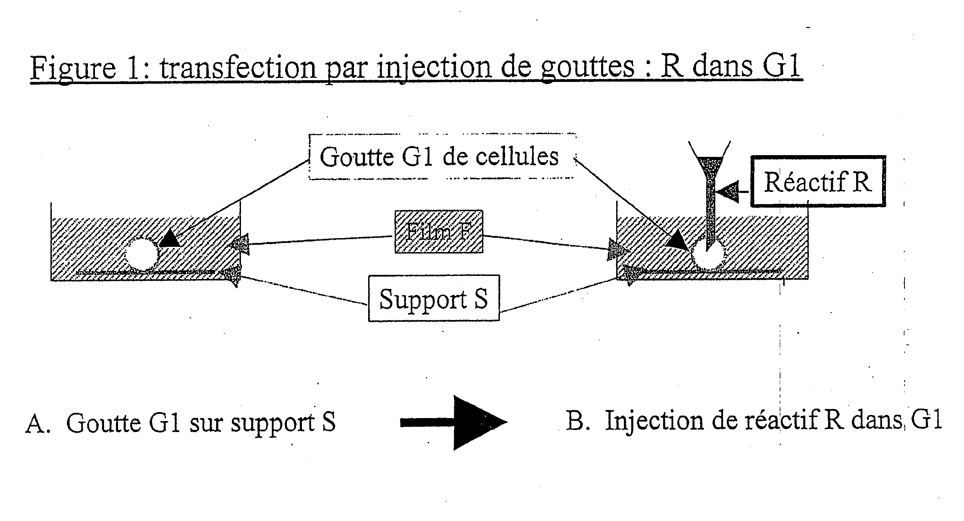

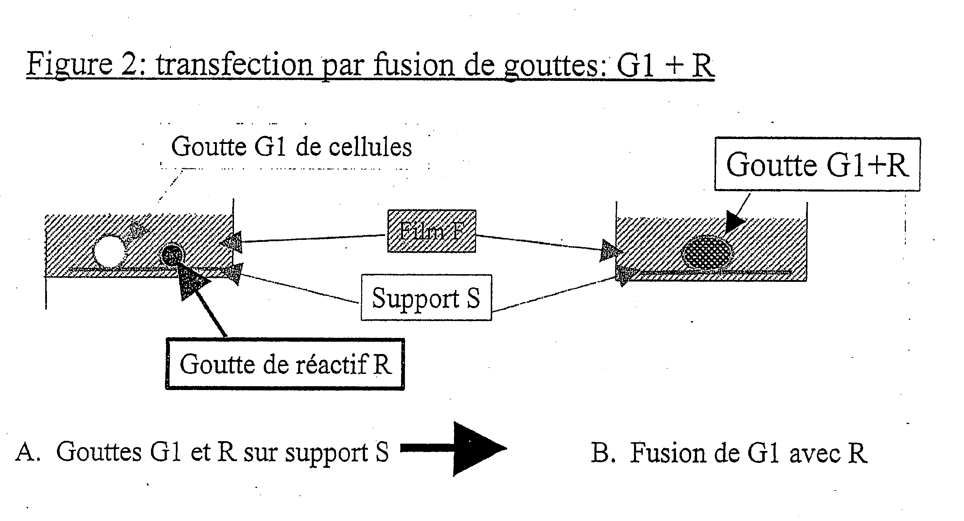

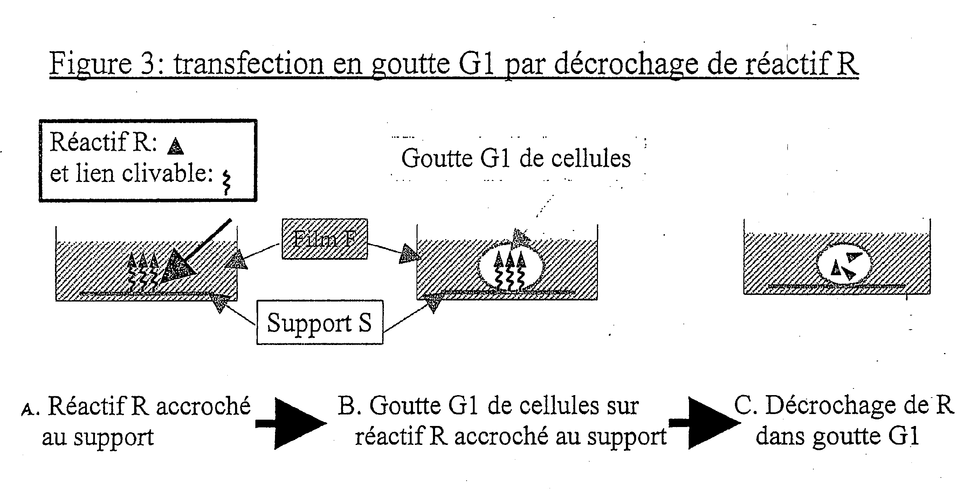

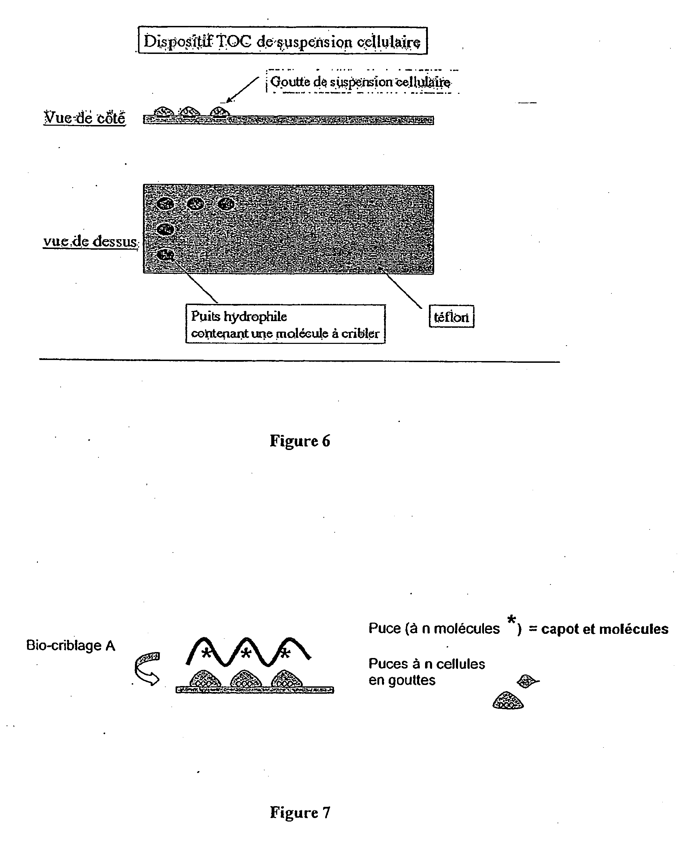

Plusieurs variantes existent pour l'introduction d'un réactif R dans la cellule C :
. Selon une première variante, une goutte aqueuse contenant la cellule C est déposée sur le support S, une seconde goutte aqueuse contenant le réactif R est injectée, à l'aide de tout moyen d'injection approprié, directement dans la goutte contenant la cellule C. Une telle variante est illustrée par la figure 1.
. Selon une seconde variante, une première goutte aqueuse est déposée sur le support S puis une seconde goutte aqueuse est déposée sur le même support à proximité de la première, l'une de ces gouttes contient la cellule C, l'autre le réactif R, la réaction du réactif R avec la cellule C, et éventuellement sa transfection dans la cellule C, est déclenchée par la fusion des deux gouttes. Le déplacement et la fusion des gouttes peuvent être obtenus par vibration au sein du support, par déplacement électrophorétique des gouttes chargées électriquement ou par des pinces mécaniques ou optiques. Il peut également être obtenu par une modification des propriétés de surface du support provoquées par l'application d'un champ électrique, magnétique , par un traitement thermique ou optique approprié. Une telle variante est illustrée par la figure 2.
. Selon une troisième variante, le réactif R est fixé au support S ou au film F, la cellule C est déposée sous forme d'une goutte aqueuse sur le support S et le réactif R est alors décroché du support S ou du film F afin de permettre sa réaction avec la cellule et éventuellement sa transfection dans la cellule. Cette variante est illustrée par les figures 3 et 7.

Dans la présente invention, le terme de transfection est utilisé pour désigner la pénétration d'une molécule d'un réactif, quel qu'il soit, dans une cellule.

Dans le cas où la stimulation du milieu réactionnel comporte l'introduction d'une molécule d'un réactif R, le film de séparation F, lorsqu'il est présent, est choisi de façon à être non miscible avec R.

Parmi les moyens d'apport d'énergie, on peut citer en particulier des moyens de traitement thermique, qui peuvent consister par exemple en un dispositif chauffant susceptible d'être placé à proximité du support S ou fixé à ce support et destiné à porter les gouttelettes à une température appropriée. Par exemple, le moyen de chauffage peut consister en des fils conducteurs de l'électricité servant également de moyen de réception des gouttes.

Les moyens de traitement optique sont notamment des moyens de traitement aux rayons ultra-violets, ces derniers étant connus pour induire une réticulation entre des brins complémentaires d'ADN et entre ADN et protéines.

La mise en contact avec une ou plusieurs autres cellules consiste à introduire dans le milieu réactionnel une ou plusieurs autres cellules de façon à constituer un réseau de cellules susceptible d'interagir.
. Selon une première variante, une goutte aqueuse contenant la cellule C est déposée sur le support S, une seconde goutte aqueuse contenant une ou plusieurs autres cellules est injectée, à l'aide de tout moyen d'injection approprié; directement dans la goutte contenant la cellule C. L'ordre de dépôt des gouttes de cellules peut bien évidemment être inversé.
. Selon une seconde variante, une première goutte aqueuse est déposée sur le support S puis une seconde goutte aqueuse est déposée sur le même support à proximité de la première, l'une de ces gouttes contient la cellule C, l'autre goutte contenant une ou plusieurs autres cellules, l'interaction entre les cellules est déclenchée par la fusion des deux gouttes. Le déplacement et la fusion des gouttes peuvent être obtenus par vibration au sein du support, par déplacement électrophorétique des gouttes chargées électriquement ou par des pinces mécaniques ou optiques. Il peut également être obtenu par une modification des propriétés de surface du support provoquées par l'application d'un champ électrique, d'un champ magnétique, d'un traitement thermique, d'un traitement optique.

Préférentiellement, le support S est constitué par une plaque qui peut être en silicium, en verre ou en polymère, comme par exemple en polyuréthane, en nylon, en polyester, en polyéthylène, en polypropylène, en polyfluorocarbone, en polyméthylméthacrylate (PMMA), en polycarbonate, en chlorure de polyvinyle (PVC), en polydiméthylsiloxane (PDMS) ou en polysulfone.

Selon l'invention, l'accrochage des gouttes sur le support se fait grâce aux forces de tension de surface. Préférentiellement, le support S présente une surface sensiblement plane comportant au moins un moyen destiné à la réception des gouttes aqueuses.

De préférence, le moyen destiné à la réception des gouttes aqueuses consiste en des zones de la surface sensiblement plane du support S d'une taille allant de 5 µm² à 5 mm².

Selon une première variante, on peut prévoir que le support S présente sur sa surface plane un caractère hydrophobe et comporte une ou plusieurs zones hydrophiles constituant ledit moyen de réception. Selon une autre variante, on peut également prévoir que le support S comporte sur sa surface plane des cavités, d'une profondeur allant de 1 micron à 1 millimètre, et constituant ledit moyen de réception. On peut également prévoir que le support S soit une plaque munie d'excroissances de faible épaisseur, de 1 micron à 1 millimètre, disposées sur sa surface et destinées à favoriser l'accrochage des gouttes. Enfin, on peut prévoir que le support S soit une plaque munie d'au moins un fil, sur lequel viennent s'accrocher les gouttes. Le dépôt de deux gouttes sur le même moyen de réception va favoriser la fusion de ces deux gouttes et donc la réaction du réactif R avec la cellule C. De préférence, le support S présente sur sa surface plane un caractère hydrophobe et comporte-une ou plusieurs zones hydrophiles constituant le moyen de réception. Pour conférer à la surface plane du support un caractère hydrophobe, celle-ci est preférentiellement recouverte d'un matériau hydrophobe tel qu'un polyfluorocarbone, comme par exemple le polytétrafluoroéthylène ou Téflon^{®}, un silane comme par exemple le perfluorosilane. La zone hydrophobe du support peut consister en une structuration de surface dentelée à l'échelle nanométrique comme le « silicium noir » utilisé en optique. Des exemples de lames commerciales de ce type sont les lames immunofluorescence 40 puits D2 mm super téflon, commercialisées par la société MERCK EUROLAB division POLYLABO. Encore plus préférentiellement, le support comporte en outre un second moyen de réception des gouttes superposé au premier, comme par exemple une surface plane hydrophobe et des excroissances de faible épaisseur hydrophiles, ou une surface plane hydrophobe et des puits hydrophiles comme illustré par la figure 6, ou une surface plane hydrophobe et un fil hydrophile.

Le support peut-être adapté à partir d'un support utilisé classiquement pour la spectrométrie de masse, on peut prévoir qu'il comporte une couche électriquement conductrice (acier par exemple) ou non ; qu'il soit recouvert, au moins sur les moyen de réception d'un matériau promoteur de la désorption. De tels supports existent sur le marché, par exemple :
- les puces SELDI (« ProteinChip^{®} arrays ») commercialisées par la société Ciphergen Inc. (modèle NP20 par exemple) comportent une fine couche hydrophobe percée de trous où la surface est active (hydrophile) ;
- les supports « AnchorChip^{™} » commercialisés par la société BrukerDaltonics Inc comportent des surfaces hydrophobes sur laquelle sont placées des excroissances hydrophiles.

Le support peut aussi être actif, pour faire évoluer les gouttes sur sa surface en utilisant les principes de la microfluidique en goutte. Cela revient à modifier dynamiquement les propriétés de surface du support (par exemple les variations en énergie/tension de surface) pour faire bouger les gouttes de manière contrôlée. Ainsi, les gouttes de cultures cellulaires peuvent passer par différentes étapes de réactions conduites au sein du support : on peut faire fusionner deux gouttes qui se rapprochent (une avec le réactif et une autre avec les cellules par exemple).

Pour réaliser ce genre de support Shenderov et al. (« Electrowetting-based actuation of liquid droplets for microfluidic applications", Applied Physics Letters, vol. 77, n°11, p. 1725-1726, septembre 2000) décrit l'utilisation de la modification des énergies de surface d'une couche hydrophobe, lors de l'application d'un champ électrique : la tension de surface diminue avec l'intensité du champ, la surface devient moins hydrophobe, voir hydrophile. Le contrôle et le mouvement du champ électrique permettent de déplacer les gouttes de liquides sur cette surface. Cette méthode a été brevetée par la société Nanolytics (Shenderov *et al.* « Actuators for microfluidics without moving parts », n° US 6,565,727 ; 2003), mais sans l'utilisation de cultures cellulaires.

Une autre manière de modifier ces propriétés de surface consiste en la modification physico-chimique de la couche surfacique du support, toujours à l'aide d'un potentiel électrique. Par exemple, le changement de conformation d'une couche de SAM (« self-assembled monolayer », monocouche auto-assemblée, par exemple des thiols modifiés, comprenant au moins une extrémité hydrophile et une chaîne hydrophobe), démontré par Lahann et al. (« A reversibly switching surface », Science, vol. 299, p. 371-374, janvier 2003), permet de passer d'une conformation droite des molécules au sein de la couche superficielle, qui alors présente un caractère hydrophile, à une conformation courbée, dans laquelle elle présente un caractère hydrophobe.

De la même manière, on peut utiliser la température comme moyen de changer les propriétés de surface d'un support. Liang et al. ("Preparation of Composite-Crosslinked Poly (N-Isopropylacrylemide) Gel Layer and Characteristics of Reverse Hydrophilic-Hydrophobic Surface" Journal of Applied Polymer Science 72:1, 1999) décrit un polymère qui est hydrophile à faible températures (<30°C) et hydrophobe au-dessus. En intégrant un système de contrôle localisé de la température sous le substrat, on peut contrôler les propriétés de la surface.

On peut aussi mettre en place un support dont les propriétés de la couche surfacique change avec l'application ou non de lumière (champs électromagnétique). Ichimura et al. (« Light-driven motion of liquids on a photoresponsive surface », Science, vol. 288, p. 1624-1626, juin 2000) décrit une telle surface : une couche de polymère (calix[4]resorinarene) dont le groupe terminal (azobenzene) peut changer de conformation isomérique après une photoirradiation asymétrique. Lorsqu'on expose ces groupes cycliques en conformation *trans* (couche hydrophile) aux UV (365nm), ils passent en conformation *cis* (hydrophobe). La réaction est réversible en utilisant de la lumière bleue (436 nm). En éclairant de façon sélective et graduelle la couche de polymère, on peut déplacer des gouttes de liquide de manière contrôlée.

Selon une variante de l'invention, le réactif R est fixé au support S avant le dépôt de la goutte aqueuse contenant la cellule C. De tels dispositifs sont connus de l'homme du métier pour d'autres utilisations : ce sont les puces à ADN telles que décrites par:
- Eisen M.B., Spellman P.T., Brown P.O., Botstein D. Cluster analysis and display of genome-wide expression patterns, Proc Natl Acad Sci USA. 1998 Dec. 8; 95(25): 14863-8;
- Haab B.B., Dunham M.J., Brown P.O., Protein microarrays for highly parallel detecting and quantitation of specific proteins and antibodies in complex solutions, Genome Biol. 2001 Jan 22; 2(2): RESEARCH 0004.1-0004.13;
- Livache T., Bazin H., Caillat P., Roget A., Electroconducting polymers for the construction of DNA or peptide arrays on silicon chips, Biosens Bioelectron. 1998 Sep 15;13(6):629-34.

On peut appliquer le même principe à des molécules autres que des polynucléotides. Des puces à molécules sont décrites dans : Kuruvilla et al., Glucose signalling with small molecule microarrays, Nature (2002), 416 p. 653. Dans tous les cas, la molécule de réactif est d'abord accrochée sur la puce (par exemple par accrochage covalent sur une lame de verre). Selon la présente invention, la molécule peut éventuellement être décrochée après dépôt des gouttes aqueuses contenant des cellules sur la puce à molécules.

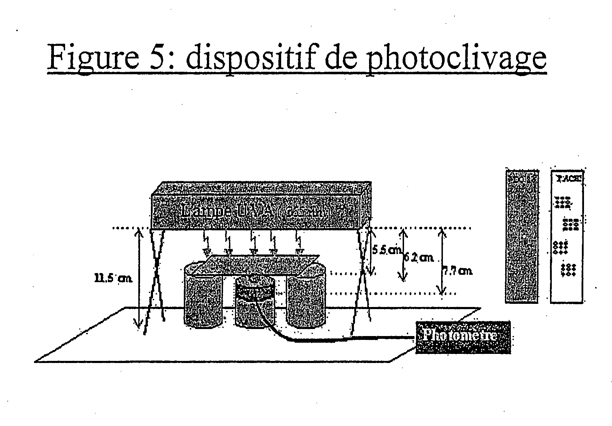

Le décrochage de la molécule de réactif peut être fait de façon connue par l'un des moyens suivants :
- photoclivage par des UV en utilisant un site de liaison du réactif au support qui soit photoclivable comme illustré par la figure 5.

Et dans le cas où le réactif est un polynucléotide uniquement :
- coupure de l'ADN double brin par des enzymes de restriction, ou par d'autres nucléases,
- modification de la stringence d'hybridation : un changement de concentration saline, de température ou de conditions Redox du milieu permet de séparer deux brins d'ADN.

Dans certains cas on prévoit que le réactif R reste fixé sur le substrat.

Selon l'invention, la surface sensiblement plane du support S peut être recouverte d'un film de séparation qui remplit trois fonctions :
- il doit prévenir une fusion des gouttes aqueuses non désirée,
- il empêche l'évaporation des gouttes aqueuses déposées sur le support,
- il permet le passage des gaz, notamment O₂ et CO₂ ces deux dernières fonctions étant destinées à permettre la survie des cellules dans leurs gouttes.

Le film F peut être de différentes natures :
- il peut s'agir d'un liquide non miscible avec l'eau comme par exemple une huile. Jusqu'à ce jour, on savait utiliser l'huile pour conserver certaines cellules, toutefois, elle n'avait jamais été utilisée pour effectuer des réactions sur des cellules. Parmi les huiles utilisables dans le procédé et le dispositif selon l'invention, on peut citer notamment les huiles minérales et les huiles de silicone. On peut également utiliser comme liquide un solvant organique non miscible avec les composés à traiter (cellules et réactifs), tel que par exemple l'octane. De préférence on utilise une huile minérale légère.
- il peut également s'agir d'un gaz comme de l'air saturé en humidité,
- il peut ensuite s'agir d'un film souple, solide, tel qu'un film en PDMS (polydiméthyl siloxane) ou un film en nitrocellulose,
- il peut enfin s'agir d'un capot rigide alvéolé en matériau poreux, la taille des alvéoles étant adaptée pour pouvoir contenir la goutte de cellule(s) et éventuellement de réactif. Selon une variante de l'invention, le capot rigide alvéolé peut être fonctionnalisé, dans chaque alvéole, par une molécule de réactif et constituer ainsi une puce à molécule ou une puce à nucléotide appelée à venir en contact avec le support sur lequel des gouttes de cellules ont été déposées de façon symétrique par rapport aux alvéoles. Cette variante de l'invention est illustrée par la figure 7.

Avant introduction du milieu réactionnel à analyser dans le spectromètre de masse, le film de séparation, s'il est susceptible d'être gênant pour la mise en oeuvre des étapes de cette analyse, est retiré.

Lorsqu'il n'y a pas de film de séparation ou que celui-ci est un gaz ou un liquide, de façon avantageuse, le dépôt des gouttes aqueuses contenant une cellule ou un réactif sur le support S, et éventuellement sous le film de séparation, se fait au moyen de fins capillaires, comme illustré sur la figure 1. De préférence, ces capillaires sont reliés à une pompe ou pousse-seringue permettant de contrôler le volume des gouttes.

Les cellules et les réactifs peuvent être également dispensés par un système classique comme ceux utilisés pour la fabrication des puces à ADN. On peut citer par exemple les systèmes piézoélectriques permettant de comprimer une cavité et d'éjecter une goutte par une buse. On peut se reporter à ce sujet à N. Takada et al., Proceeding of the SID, vol. 27/1, 1986, 31-35.

Préférentiellement, les gouttes éjectées passent à travers le film de liquide ou de gaz grâce à leur vitesse d'éjection et/ou par gravité, ce liquide ou ce gaz étant plus léger que la solution à déposer. Lorsque le film de séparation est un film solide ou un capot rigide, celui-ci est déposé sur le support, après dépôt des gouttes aqueuses de cellules et éventuellement de réactifs par les mêmes moyens que décrit ci-dessus.

Le déplacement et la fusion des gouttes peuvent être obtenus par vibration au sein du support, par déplacement électrophorétique ou électromagnétique des gouttes chargées électriquement ou par des pinces mécaniques ou optiques. Il peut également être obtenu par une modification des propriétés de surface du support provoquées par l'application d'un champ électrique, d'un champ magnétique, par un traitement thermique ou un traitement optique..

Préférentiellement, le support S du dispositif est mobile, de façon à permettre son déplacement d'un premier moyen de dépôt à un second moyen de dépôt, et éventuellement à d'autres moyens de dépôt et également le déplacement jusqu'au spectromètre de masse. Le support S peut dans certains cas être constitué d'un film solide fixé à des rouleaux à ses deux extrémités, les rouleaux étant munis de moyens de bobinage de façon à permettre le déplacement du film et donc le déplacement des gouttes qui ont été déposées dessus.

Généralement, le procédé selon l'invention prévoit le déplacement du support S après le dépôt sur le support S de la première série de gouttes, qu'il s'agisse des gouttes de cellules ou des gouttes de réactif.

Selon l'invention le support S peut être placé dans une enceinte à atmosphère contrôlée dont la température, l'hygrométrie et la teneur en CO₂ sont ajustées de façon à permettre la survie des cellules.

De tels dispositifs sont notamment des étuves à atmosphère contrôlée. La température dans un tel dispositif peut varier de 35 à 42°C, une température préférée se situant entre 36,5 et 37,5°C. La variation de température peut notamment être utilisée pour induire des différentiations cellulaires.

Le taux de CO₂ est préférentiellement maintenu entre 3 et 5%. Le taux d'oxygène O₂ est préférentiellement celui de l'air ambiant.

Par exemple, on peut prévoir de maintenir les cellules dans des gouttes aqueuses sur le support S dans une étuve à 37°C, avec 95% d'air, 5% de CO₂ et 97% d'humidité.

On prévoit généralement que seuls les supports sur lesquels ont été déposées les gouttes de cellules et le film de séparation soient placés dans une enceinte à atmosphère contrôlée. Toutefois, d'autres éléments du dispositif de l'invention peuvent si nécessaire être placés dans cette enceinte.

De façon avantageuse, on peut prévoir que les gouttes aqueuses contenant une ou plusieurs cellules, un tissu cellulaire ou un réseau cellulaire, comportent un milieu de culture.

En effet, l'établissement de cultures de cellules dépend de l'aptitude des cellules à maintenir leur prolifération et donc des conditions indispensables à leur croissance.

Avantageusement, on prévoit que les gouttes aqueuses de cellules comprennent du MEM ou « minimal essential medium » commercialisé par la société GIBCO BRL sous la référence Cat. No. 12000-022.

Le milieu de culture peut également contenir d'autres constituants tels que du sérum de veau, un ou plusieurs antibiotiques destinés à contrôler la stérilité du milieu, comme par exemple de la pénicilline.

On peut également prévoir d'utiliser dans le milieu de culture des agents chimiques qui induisent la différentiation des cellules, comme par exemple la bromodéoxyuridine.

On peut également prévoir que les gouttes aqueuses dans lesquelles les cellules C sont en culture soient gélifiées, à l'aide de tout agent gélifiant connu, comme par exemple de l'agar ou de la gélatine.

De façon avantageuse, les gouttes aqueuses contenant la ou les cellules ou le tissu cellulaire ou le réseau de cellules, et/ou les gouttes aqueuses contenant le réactif, comportent un ou plusieurs constituants destinés à favoriser la transfection, comme par exemple des liposomes. De tels agents de transfection sont décrits notamment dans les documents WO 01/20015 et WO 98/33932.

D'autres moyens destinés à favoriser la transfection peuvent également être employés dans le dispositif de l'invention, tels que : l'électroporation ou la microprécipitation. Ces méthodes de transfection, bien connues de l'homme du métier, sont décrites notamment sur http://opbs.okstate.edu/~melcher/MG/MGW4/MG43.html.

On peut en outre prévoir que le dispositif comporte des moyens de détection focalisés sur une ou plusieurs gouttes déposées sur le support.

Les moyens de détection sont notamment des dispositifs destinés à mesurer la fluorescence ou la radioactivité d'une ou plusieurs gouttes ou des cellules contenues dans une ou plusieurs gouttes.

Les moyens utilisés dans les dispositifs selon l'invention seront préférentiellement reliés à un dispositif de contrôle permettant l'automatisation du dispositif et du procédé selon l'invention.

L'utilisation du dispositif et/ou du procédé selon l'invention présente de nombreux avantages : on peut utiliser de très petites quantités de matériaux : une seule cellule par goutte permet de réaliser une expérience de transfection et suffit pour faire une analyse par spectrométrie de masse. On peut travailler avec des volumes de gouttes très petits, inférieurs à 1 micro litre, de préférence de 0,1 à 1000 nanolitres contenant 1 à 500 cellules, encore plus préférentiellement de 0,1 à 10 nanolitres contenant 1 à 10 cellules. Avantageusement, on travaille sur des gouttes contenant de 1 à 100 cellules. On peut également prévoir de travailler sur des volumes plus importants, notamment supérieurs au microlitre (10 à 100µl, contenant 500 à 100 000 cellules). Ce procédé permet également d'utiliser de petites quantités de réactif. Le film de séparation F. permet de contrôler les échanges gazeux du milieu de culture de la cellule et sa stérilité. Il permet aussi de séparer des gouttes dont on ne souhaite pas qu'elles régissent ensemble. Les cultures de cellules sous forme de gouttes sous le film de séparation peuvent se conserver au moins 24 heures et jusqu'à plusieurs jours sans que l'on observe de modifications notables de leur activité cellulaire (sans influence notable sur la prolifération et la croissance des cellules).

Le procédé et le dispositif selon l'invention permettent également de réaliser des batteries de réactions:
Plusieurs gouttes aqueuses comprenant chacune au moins une cellule peuvent être déposées sur le support S, lesdites gouttes étant isolées les unes des autres. De préférence, chacune de ces gouttes est placée dans un moyen de réception distinct. Toutes les cellules peuvent être identiques, mais on peut également prévoir de placer des cellules différentes (au moins deux sortes de cellules différentes) dans les différentes gouttes. Des gouttes contenant le ou les réactifs sont déposées, à proximité de chaque goutte contenant une cellule, de façon à permettre la fusion d'une goutte contenant le réactif approprié avec la goutte contenant la cellule visée. On peut également prévoir d'injecter une goutte de réactif directement dans chaque goutte de cellule. Pour la réalisation de batteries de réactions, on prévoit avantageusement un support comprenant des moyens de réception des gouttes disposés de façon régulière sous forme de matrices, de façon à permettre l'automatisation du procédé.
De façon avantageuse, le support et les capillaires destinés à déposer les gouttes aqueuses de cellules et de réactifs sont reliés à des moyens de contrôle de façon à permettre l'automatisation du procédé.
Le procédé et le dispositif selon l'invention permettent donc de réaliser simultanément et de façon automatisée un grand nombre de réactions d'un réactif sur une cellule en faisant varier la nature du réactif et de la cellule, tout en travaillant sur des volumes extrêmement réduits, puis d'analyser le résultat de ces réactions.

Parmi les cellules qu'il peut être intéressant d'étudier par ce procédé, on peut citer notamment :
- des cellules primaires,
- des hybridomes,
- des lignées de cellules : les cellules peuvent se perpétuer éternellement et former ainsi des lignées,
- des cellules souches : elles sont obtenues à partir d'un prélèvement chez l'animal ou à partir de biopsies,
- un morceau de tissu cellulaire (les cellules ne sont pas individualisées)
- des mélanges des différents types de cellules énoncés ci-dessus.

Les cellules sont cultivées en milieu de culture (aqueux) de façon connue. On peut également cultive des cellules hétérogènes pendant plusieurs jours et utiliser ce mélange.

Selon une variante-de l'invention, lorsque toutes les cellules à mettre en réaction sur un même support sont identiques on peut procéder de la façon suivante : le support est une plaque hydrophobe comportant des zones hydrophiles, on l'immerge dans une solution aqueuse contenant les cellules puis on la sort de cette solution en laissant le liquide en excès s'écouler. Les gouttes du milieu contenant les cellules sont retenues dans les zones hydrophiles. Cette étape est suivie du dépôt d'une couche de film de séparation F et du dépôt des gouttes contenant un réactif ou d'autres cellules. Suivant la nature du film F (fluide ou solide), celui-ci est déposé avant ou après le dépôt des gouttes de réactif ou d'autres cellules.

Parmi les réactifs R susceptibles d'être utilisés dans le procédé et le dispositif selon l'invention, on peut citer :
Des molécules chimiques de toutes natures, notamment des molécules inorganiques, des molécules organiques naturelles, des molécules issues de synthèse organique et de synthèse combinatoire, des molécules extraites d'échantillons biologiques et des molécules extraites d'échantillons biologiques modifiées par synthèse. On peut citer notamment les polynucléotides : les molécules d'ARN simple et double brin, les molécules d'ADN simple et double brin ; les molécules de PNA (de l'anglais « peptidic nucleic acid » ou acide nucléique peptidique) qui sont des chimères peptide-acide nucléique ; les ribozymes ; les ARN interférences double brin ou les protéines et les peptides. Parmi les protéines, on peut citer tout particulièrement les facteurs de transcription.

Les molécules de réactif peuvent être formulées en solution prête à être déposée. Elles peuvent également être préparées directement après dépôt sur le support, par exemple par synthèse, notamment par synthèse organique, *in situ,* ou par transcription *in vitro* dans la goutte. Des molécules de type prion peuvent aussi être obtenues en goutte par réaction de polymérisation en chaîne ou PCR (de l'anglais "polymerisation chain reaction") peptidique avant leur transfection dans les cellules. Lorsque l'on utilise des molécules d'acides nucléiques, leur préparation peut être faite par PCR nucléique. Comme il a déjà été exposé ci-dessus, le réactif peut également être fixé au support.

Lorsque l'on emploie comme réactif de l'ADN, avantageusement celui-ci est sous forme précipitée. De façon connue, on peut par exemple utiliser le phosphate de calcium. La précipitation de l'ADN peut également être faite dans la goutte aqueuse déposée sur le support par fusion avec une goutte du réactif approprié.

Selon une variante du dispositif et du procédé selon l'invention, on peut prévoir de faire plusieurs dépôts successifs destinés à être fusionnés :
On peut prévoir de déposer successivement plusieurs réactifs destinés à transfecter une même cellule et observer leurs effets cumulés ;

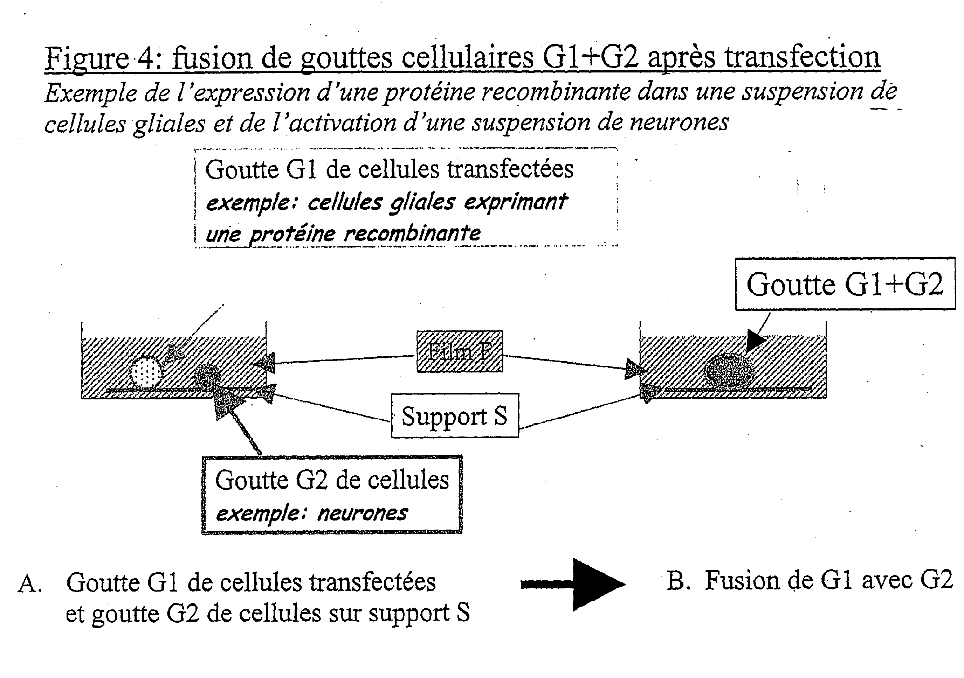

On peut également prévoir de déposer plusieurs gouttes de cellules et les faire fusionner; de façon à reconstituer un réseau cellulaire de cellules identiques ou différentes afin de se rapprocher au plus près de conditions rencontrées *in vivo.* Par exemple, on peut reconstituer des réseaux de neurones à l'échelle de quelques cellules par la rencontre de cellules gliales et de neurones pour les faire communiquer au sein d'une même goutte comme illustré par la figure 4, ou des interactions entre les différents types de cellules qui constituent la peau afin de mimer le comportement de celle-ci à l'échelle cellulaire.

On peut également reconstituer un tissu cellulaire destiné à mimer le comportement de l'épiderme en cultivant ensemble au sein d'une même goutte des kératinocytes sur un tapis de collagène. On peut également cultiver ensemble des cellules souches de la peau en présence de cellules du follicule pilleux afin d'étudier leurs interactions.

Par exemple, on peut utiliser la transfection de réactifs dans un premier type de cellules de façon à déclencher une réaction cellulaire, telle que la production d'une protéine recombinante puis faire réagir cette première population cellulaire avec une population cellulaire d'un autre type par fusion avec une autre goutte.

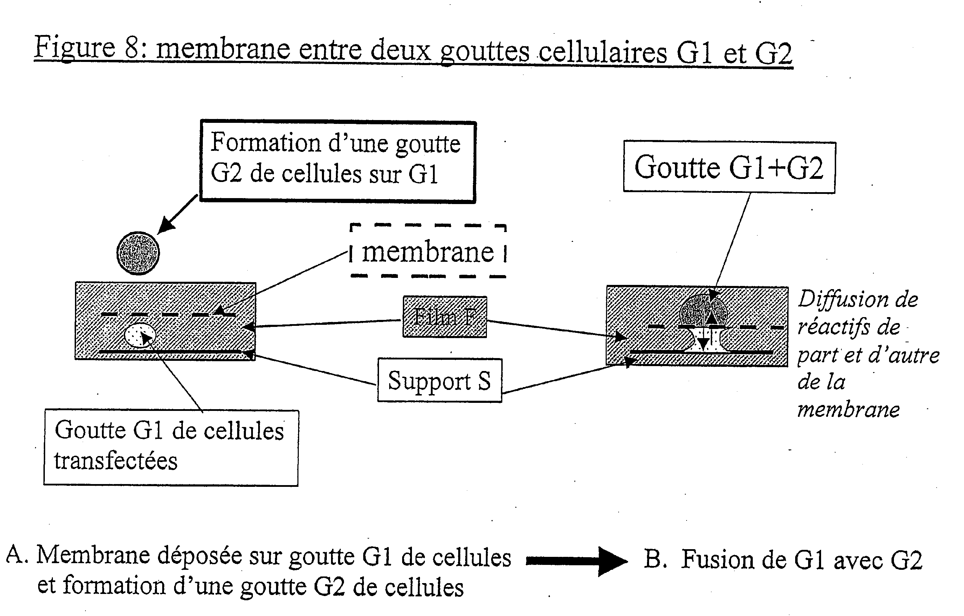

Selon une variante de l'invention illustrée par la figure 8, on peut également prévoir que le support soit muni de moyens de séparation permettant de séparer deux types de cellules distincts mais autorisant le passage de petites molécules entre ces cellules. Un tel moyen de séparation est destiné à mimer une barrière biologique, telle que par exemple la barrière existant entre le sang et les cellules cervicales. De tels moyens de séparation sont avantageusement disposés au niveau des moyens de réception, sur le support. Pour leur mise en oeuvre, on prévoit de déposer une goutte aqueuse comprenant au moins une cellule d'un premier type d'un coté du moyen de séparation et une goutte aqueuse comprenant au moins une cellule d'un second type de l'autre côté du moyen de séparation. La fusion des gouttes de part et d'autre du moyen de séparation permet une communication entre les cellules par l'intermédiaire de molécules susceptibles de diffuser au travers du moyen de séparation. Cette communication peut alors être étudiée par tous moyens, notamment par l'ajout de réactifs sous forme de goutte aqueuse avant ou après la fusion des gouttes cellulaires. Par la transfection automatisée de ces gouttes, il est possible d'analyser le rôle biologique des facteurs transfectés dans une multicouche biologique.

Les moyens de séparation utilisables selon cette variante de l'invention sont des membranes artificielles telles que par exemple un filtre en nitro-cellulose, du silicium percé de nano-trous, un buvard en papier, un filtre en tissu; on peut également prévoir d'utiliser un gel solide tel qu'un gel d'agarose, du collagène ou de la gélatine.

Le dispositif et le procédé selon l'invention permettent d'étudier l'expression automatisée de protéines recombinantes obtenues par l'entrée d'ADN codant dans les cellules, de réaliser le criblage de molécules nucléiques destinées à modifier (bloquer ou au contraire augmenter) l'expression de gènes dans les cellules et de rechercher des séquences génomiques promotrices. Cette invention permet aussi d'étudier les interactions entre des cellules de différents types, cette interaction étant déclenchée par le mélange des gouttes. Le dispositif et le procédé selon l'invention permettent d'obtenir une vue globale des effets biologiques de la réaction de molécules de toutes sortes avec des cellules, et notamment de l'entrée automatisée de molécules de toutes sortes dans des cellules.

Selon une variante de l'invention, on peut prévoir une ou plusieurs étapes de traitement du milieu réactionnel directement sur le support S avant son introduction dans le spectromètre de masse. Ces étapes de traitement peuvent consister en une lyse des cellules, un ou plusieurs lavages, l'adsorption ou la fixation de molécules ayant une affinité pour des molécules dont on cherche à détecter la présence.

Ensuite, le milieu réactionnel placé sur le support S est préparé en vue de son introduction dans le spectromètre de masse.

Cette préparation peut consister en une congélation du milieu réactionnel de façon à préserver ses caractéristiques. Elle peut consister en un séchage, avec ou sans traitement thermique, avec ou sans vide, comme par exemple par lyophilisation. On peut aussi prévoir de les fixer par un traitement à l'aide d'un agent tel que le méthanol ou le formaldéhyde. On peut prévoir d'appliquer plusieurs étapes de préparation successives.

Selon le procédé de l'invention, lorsque le spectromètre de masse est du type MALDI la préparation du milieu réactionnel en vue de son introduction dans le spectromètre de masse comporte de façon connue l'addition d'une ou de plusieurs molécules acides, de petite taille et absorbant la lumière telles que par exemple l'acide alpha cyano 4-hydroxycinnamique, l'acide nicotinique ou l'acide sinapinique. Ladite molécule en solution est additionnée au milieu réactionnel à analyser de façon à ce qu'après séchage, l'échantillon à analyser soit inclus dans la matrice cristalline formée par cette molécule, ce qui permet d'assurer le succès de la désorption et de l'ionisation de l'échantillon.

La solution de molécule destinée à former la matrice cristalline est avantageusement additionnée au milieu réactionnel en très large excès molaire de façon à favoriser la formation de la matrice cristalline lors du séchage à l'air de l'ensemble. Lorsqu'un autre type de spectromètre est employé, on utilise d'autres molécules promotrices de la désorption, de façon connue de l'homme du métier.

Le milieu réactionnel est alors désorbé et ionisé. Cette étape est mise en oeuvre grâce à un moyen de désorption sélectionné parmi : un faisceau laser, un faisceau d'ions, un faisceau d'atomes neutres, un faisceau d'électrons. La désorption/ionisation peut également être faite par nébulisation d'un échantillon liquide. La résolution est de l'ordre de la taille du faisceau et permet un ciblage très précis du milieu réactionnel que l'on souhaite désorber. Lorsqu'une pluralité de milieux réactionnels se trouve sur le support S, chacun des dépôts peut être ciblé, et donc désorbé/ionisé, de façon successive.

Chacun des milieux réactionnels déposés sur le support S est désorbé et ionisé individuellement et son spectre de masse est également produit de façon individuelle et ciblée.

Le support S est placé dans le spectromètre de masse et chacun des milieux réactionnels placés sur ce support est traité de façon individuelle. Un même milieu réactionnel peut être traité à plusieurs reprises (plusieurs milliers de traitements laser) pour donner une analyse plus complète du phénotype considéré.

La disposition des dépôts sur le support S sous forme de matrice permet une automatisation de la désorption et de l'ionisation.
- On peut citer les appareillages suivants connus comme moyens de désorption/ionisation :
   o MALDI : matrix assisted laser desorption ionisation (désorption et ionisation au laser assisté par la présence d'une matrice) et son pendant le
   o SELDI, surface enhanced laser desorption ionisation (désorption et ionisation améliorées par une surface active)
   o SIMS : secondary ion mass spectrometry (spectrométrie de masse à ions secondaires)
   o SNMS : secondary neutral mass spectrometry (spectrométrie de masse à neutres secondaires)
   o ESI : electrospray ionisation (ionisation par nébulisation)
   o FAB : fast atom bombardment (bombardement d'atomes rapides)
   o APCI : atmospheric pressure chemical ionisation (ionisation chimique à pression atmosphérique)
- On peut citer les appareillages suivants connus comme moyens de mesure de la masse :
   o TOF : time of flight, temps de vol
   o MS/MS (ou tandem mass spectrometry ou multi-dimensionnal MS pour MS/MS/MS/etc...) : spectrométrie de masse en tandem, spectrométrie de masse multidimensionnelle.
   o Quadrupole (ou ion trap) : quadrupole ou trappe à ion
   o FT-MS ou FT-ICR: Fourrier-Transform mass spectrometry - ion cyclotron résonance (spectrométrie de masse à transformée de Fourrier - caractérisation par résonance cyclotronique)

Toute combinaison de ces différents moyens comportant au moins un moyen de désorption et un moyen d'analyse peut être considérée selon la présente invention comme un spectromètre de masse

Le spectre de masse obtenu pour chaque milieu réactionnel peut être comparé à une base de données de spectres de masse de façon à permettre l'identification de molécules connues au sein du milieu réactionnel.

La comparaison du spectre de masse d'une culture cellulaire avec celui d'un milieu réactionnel issu de cette culture permet d'identifier des modifications intervenues dans la culture cellulaire à la suite de la stimulation qui lui a été appliquée.

L'évolution dans le temps de la réponse d'une cellule ou d'un ensemble de cellules à une stimulation donnée peut être étudiée grâce au procédé et au dispositif de l'invention : la même stimulation peut en effet être appliquée de façon échelonnée dans le temps à une série de milieux réactionnels identiques placés en série sur un même support S.

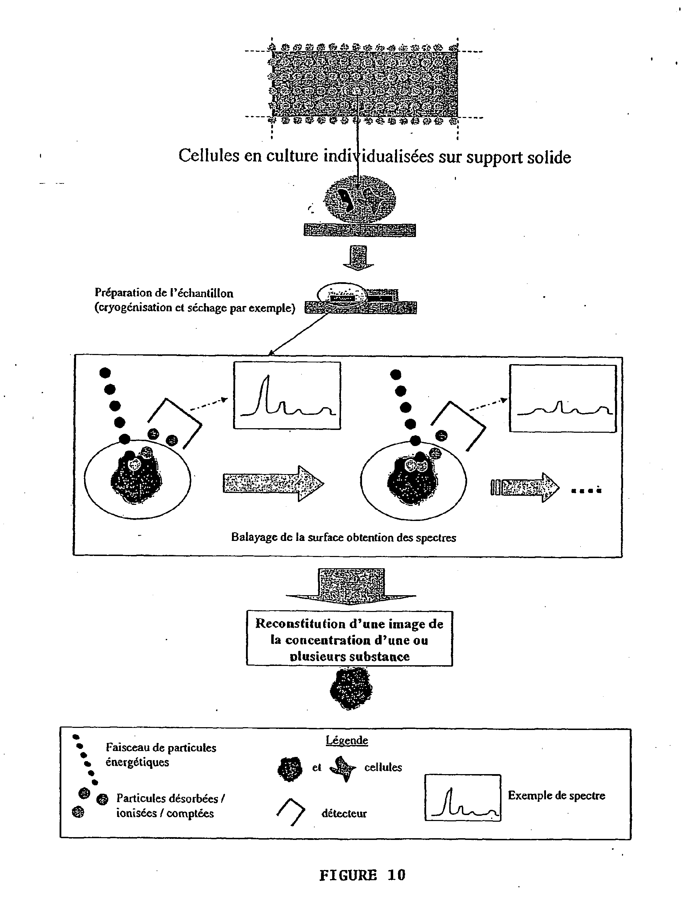

Le procédé et le dispositif de l'invention permettent de faire de l'imagerie par spectrométrie de masse : On désorbe/ionise de façon sélective sur une zone de taille réduite (en dessous de 100µm _ de l'ordre de la taille du faisceau utilisé) les molécules présentes dans l'échantillon, et ceci de façon séquentielle sur toute la surface de l'échantillon. On obtient alors pour chaque « point » de la surface, un spectre qui décrit l'abondance relative (concentration) de molécules présentes dans ce point de l'échantillon. L'abondance d'une molécule est représentée par la hauteur d'un pic (ou l'aire de la courbe en dessous de ce pic) au sein du spectre obtenu. Si l'on sélectionne un pic dans tous les spectres, on peut envisager de recréer une « image » de l'échantillon relative à cette molécule, de la même manière, que pour un scanner à fluorescence : un point est représenté par exemple par un pixel de couleur, associé à un code de couleur qui permet d'évaluer l'abondance de la molécule décrite par le pic sélectionné en ce point, l'ensemble des pixels donne une image facilement interprétable. Cette variante de l'invention est illustrée par la figure 10.

Ce processus peut être implémenté pour chacun des pics présents dans les spectres, donc à partir d'un seul balayage de la surface par le faisceau, on peut recréer une image de l'échantillon pour chaque molécule d'intérêt détectée dans l'échantillon. Une analyse par scanner à fluorescence ne permet généralement de visualiser que les molécules marquées préalablement au balayage et ce de façon séquentielle (un marquage pour une molécule et pour un balayage).

On peut aussi sélectionner plusieurs pics pour l'ensemble de l'échantillon, représentés chacun par une couleur, plus ou moins intense selon l'abondance de la molécule. La superposition des deux couleurs donne une image du ratio de concentration des substances les unes par rapport aux autres.

Le procédé et le dispositif de l'invention permettent de faire l'analyse globale d'un échantillon :
Les cultures cellulaires sont préparées pour leur introduction dans le spectromètre (séchage, cryogénisation, ajout d'une matrice promotrice de désortion/ionisation par exemple). L'échantillon est balayé de façon globale par le faisceau. On recueille un grand nombre de spectres, qui sont additionnés pour donner une moyenne de l'abondance des différentes substances au sein de l'échantillon.

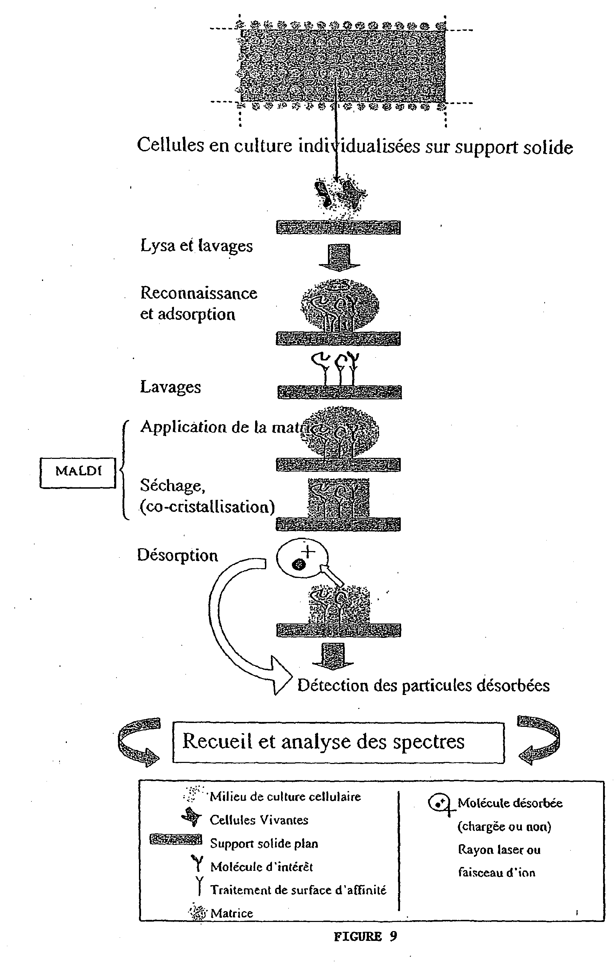

Le procédé et le dispositif de l'invention permettent de faire l'analyse globale d'un échantillon simplifié :
De manière à faciliter l'analyse du spectre obtenu lors d'un balayage global de la surface, on peut simplifier, directement sur la puce, l'échantillon avant son introduction dans le spectromètre, en utilisant des surfaces actives. Ces surfaces peuvent permettre de retenir de manière plus ou moins spécifique certaines substances de l'échantillon, à la suite de lavages comme illustré sur la figure 9.

Le procédé et le dispositif de l'invention permettent d'étudier l'évolution d'un milieu réactionnel vivant sous l'effet d'une stimulation. Cette démarche ne nécessite pas de connaître à priori les modifications (expression de protéines par exemple) engendrées par cette stimulation, comme c'est le cas pour les autres méthodes d'analyse de milieux réactionnels en matrice. En outre, toutes les réactions qui se produisent se font dans la cellule vivante, en présence des mêmes facteurs d'influence que dans un test *in vivo.* Enfin, les manipulations et traitement de purification et/ou d'extraction et/ou de transfert du milieu réactionnel peuvent être évités par le procédé de l'invention ce qui permet d'éviter les artefact et les observations biaisées qui sont propres aux systèmes d'étude *in vitro.*

De façon plus précise, le dispositif de l'invention comporte au moins un appareil permettant de mesurer la masse d'un échantillon au moyen de la spectrométrie de masse, ledit appareil comprenant un tube de spectromètre, un dispositif permettant de faire le vide dans ledit tube, des moyens électriques permettant d'appliquer un potentiel d'accélération électrique dans le tube de façon à accélérer les molécules de l'échantillon à analyser, un moyen permettant de détecter la masse des ions formés, un moyen d'introduction du support S dans le tube de façon à permettre l'introduction des milieux réactionnels associés à des molécules promotrices de la désorption dans le spectromètre, un moyen permettant la désorption et l'ionisation d'une partie des molécules de cet échantillon.

Ledit moyen permettant la désorption et l'ionisation est relié à un système d'acquisition de données de façon à permettre le traitement séquencé d'une pluralité d'échantillon à analyser placés sur un support S sous forme d'une matrice ou puce.

Le procédé de l'invention comporte les étapes suivantes :
- traitement du ou des milieux réactionnels vivants placés sur le support S par un moyen favorisant la désorption de façon à produire des échantillons prêts à être analysés ;
- introduction des échantillons (milieux réactionnels) placés sur le support S dans le tube du spectromètre de masse ; application d'un vide et d'un champ électrique dans le tube d'introduction de spectromètre de façon à former un potentiel d'accélération ; application d'un traitement de désorption/ionisation de façon contrôlée et séquencée sur le ou les échantillons ; détection de la masse des ions formés ; éventuellement comparaison des données recueillies avec une banque de spectres de masse.

Les avantages du procédé et du dispositif de l'invention sont nombreux :
Cette technologie permet en effet de réaliser des milliers de conditions expérimentales indépendantes sur un format miniature.
Le format de la micro-goutte permet un contrôle rigoureux des conditions de cultures du modèle cellulaire tout au long des différents processus mis en jeux. Ce contrôle est renforcé par l'intégration de tous les traitements des échantillons directement sur la puce à l'aide ou non de chimie de surface : culture, stimulation, purification (éventuellement).

Pour ne pas compromettre ce contrôle, comme pour toutes les méthodes existantes, aucun transfert d'échantillon n'est effectué entre les étapes de cultures et le recueil de signal final.

Ce recueil tire parti de la précision et de la spécificité de la spectrométrie de masse. Celle-ci permet aussi d'analyser un échantillon de manière multi-paramétrique (plusieurs molécules analysées en une seule expérience, voir l'ensemble d'un protéome d'intérêt) pour pouvoir s'intéresser à des systèmes sans préjuger de leur propriétés.

Cette invention, en conservant les échantillons au plus près de la culture cellulaire, permet des analyses des données recueillies beaucoup plus rapides : des milliers d'expériences multi-paramétriques sur une seule puce. Cela s'ajoute à une interprétation en terme de phénotype beaucoup plus évidente et fiable. Par exemple, aucun biais n'est introduit quant à la quantification de la présence d'une protéine (pas de marquage par exemple).

De plus en intégrant directement l'analyse par spectrométrie de masse sur la puce ou matrice contenant les cellules, l'invention tire parti de la résolution élevée de l'appareil et permet des analyses de phénotypes localisés (cartographie d'expression et imagerie cellulaire) à haut débit.

Ces analyses sont en plus quantitative, et permettent donc une étude de modulation d'expression au cours du temps.

Cette qualité d'analyse s'applique à toutes les molécules présentes dans l'échantillon cellulaire : molécules sécrétées, présentes à l'intérieur de la cellule (l'interfaçage avec un dispositif de sécrétion peut aussi être envisagé), structurelle.

La chimie de la molécule ne jouant de rôle qu'au niveau de la mise en oeuvre pratique de la spectrométrie, il est possible de détecter tout type de molécules produites par les cellules : lipides, protéines, peptides, protéines modifiées, sucres, ARN circulants.

Le phénotypage direct de cellules stimulées sur puce s'applique à tous les domaines faisant intervenir la caractérisation de réponses cellulaires :
• Recherche en biologie : les recherches cliniques et fondamentales sur les cellules et les mécanismes de fonctionnement intra ou inter cellulaires sont grandement accélérées par le recours à un outil utilisant directement des cellules et permettant un débit d'analyse élevé, couplé à une haute précision.
• Recherche pharmaceutique: en testant l'activité de nouvelles molécules (thérapeutiques, toxiques) sur des cellules vivantes de manière rapide et multi-paramétrique, le procédé de l'invention permet d'accélérer le processus de criblage, en faisant beaucoup plus tôt des tests proches de conditions *in vivo.* De plus la recherche de nouvelles cibles d'intérêt thérapeutique est aussi accélérée puisque leur pertinence est évaluée dans un environnement cellulaire complet et peu perturbé.
• Toxicologie / biocapteurs : analyse rapide de la réponse de cellules à des toxines sur de multiples paramètres (plusieurs modèles cellulaires sur une même puce par exemple) pour, par exemple, l'industrie militaire ou de l'environnement.
• Etudes des manipulations génétiques de cellules végétales ou animales.

### PARTIE EXPERIMENTALE

### Exemple 1 : Analyse des sécrétions de lymphocytes T : la cytokine IL-2.

Intérêt : Caractérisation d'échantillons de patients atteint par différents virus (Hépatite C, HIV par exemple) ou cancers. La cytokine analysée (IL-2) est un promoteur de la prolifération de lymphocytes T, elle peut être utilisée comme thérapie pour le renforcement du système immunitaire. La mesure du taux de cytokine est un indicateur de la progression de la maladie et/ou de la thérapie.

Le principe de cette expérience est d'essayer de détecter par spectrométrie de masse, la sécrétion/production de cytokine IL-2 par des lymphocytes T stimulés (lignée DO10.11, propriété de l'INSERM U548). La stimulation pouvant se faire soit directement (addition de l'antigène stimulant dans la solution), soit à l'aide de lymphocyte B présentateurs d'antigène associés (eux-mêmes stimulés pour la présentation).

Sur une puce de spectrométrie MALDI-TOF, une goutte de culture cellulaire de lymphocyte T est déposée (1500 cellules dans une goutte de 4µl de milieu RPMI + 10% SVF_ sérum de veau foetal + 1% PS). Comme contrôles/témoins, on dépose de la même façon des gouttes contenant de l'antigène seul en solution, de la cytokine seule en solution, du milieu de culture seul, des cellules non stimulées en cultures. La production de cytokine est ensuite stimulée dans les gouttes concernées.

La matrice (solution saturée d'acide sinapinique, ou d'acide alpha-cyano-4-hydroxcinnamique) est ajoutée sur chaque plot et séchée. La puce est disposée dans le spectromètre et les spectres recueillis et analysés.

Plusieurs variantes peuvent être envisagées et combinées:
- la matrice est déposée avant les cellules.
- fonctionnalisation de la surface à l'aide d'anticorps anti-cytokine, avant les dépôts, et les cellules sont lysées ou non après stimulation.
- Aucune matrice n'est déposée.

### Exemple 2 : analyse d'une signature moléculaire

Le but de cette expérience est de démontrer qu'une analyse sur un format puce d'une culture cellulaire, et plus particulièrement la distinction d'une signature moléculaire, est réalisable.

Nous avons utilisé comme échantillon une culture cellulaire (lignée jurkat, concentration 2.10⁶ cellules/ml dans leur milieu de culture RPMI+ 10%SVF -sérum de veau foetal), ainsi qu'un échantillon d'une protéine recombinante (IL2 disponible chez R&D Systems sous la référence 202-IL-050).

La puce utilisée fait partie du système ProteinChip^{®} commercialisée par la société Ciphergen Inc., utilisant le SELDI. Il s'agit d'une lame recouverte d'une couche hydrophobe percée de trous hydrophiles, où la surface active est en silice, de référence commerciale NP20, qui permet de retenir sélectivement les protéines à caractère hydrophile. Cette puce contient 8 plots notés de A à H.

Sur une première puce, on a cherché à étudier la signature spectrale de la protéine sans cellules. Pour cela on a disposé sur chaque plot une dilution particulière de la protéine allant de 5000U/ml pour le plot A à 1 U/ml pour le plot H (en passant par 2500, 1000, 500,100, 50,et 10).

Préparation de la matrice:
o Dissoudre la matrice vendue par Ciphergen (référence commerciale « EAM SPA »), acide sinapinique, dans 75 µl d'acétonitrile, et 75µl de TFA 1%.
o Vortexer la solution pendant 5 min
o Centrifuger 2min à 10 000 trs/min.

Calibrage de l'appareil :
o Déposer sur un plot de la puce un mélange de 1 µl de « All-in-one peptide mix » et 1 µl de matrice
o Laisser sécher à l'air ambiant.
o Suivre la procédure de calibration du logiciel ProteinChip^{®}.

### Préparation de la puce :

o pré-incubation de la lame avec de l'eau distillée. (5µl par plot pendant 1min)
o séchage des plots à l'aide d'un papier absorbant (sans toucher la surface)
o dépôt de 5µl d'échantillon par plot
o laisser sécher à l'air libre
o rincer à l'eau distillée (prélever à la pipette au bord du plot plusieurs fois)
o laisser sécher.
o ajouter la matrice de promotion de désorption/ionisation (0.8µl)
o laisser sécher
o réitérer l'étape d'ajout de matrice de désorption/ionisation suivie du séchage

Lecture de la puce : utilisation du système SELDI du CHU de Grenoble (inserm U318)

| | | |
|---|---|---|
| Réglages : | Intensité : | 210 |
| | High mass | 100 000 Da |
| | Déflecteur | auto (10 000 Da) |
| | Sensibilité | 10 |
| | Optimisation range : | 10 000 - 20 000 Da |
| | Center mass : | 15 400 Da |

Les spectres sont réalisés à l'aide du logiciel ProteinChip^{®} Software

Dans un deuxième temps on prépare une puce contenant les échantillons d'intérêt comme explicité dans le tableau ci-dessous :

| Plot | Echantillon |
|---|---|
| A | Calibration (1 µl « All-in-one peptide mix » + matrice |
| B | 5µl de milieu de culture sans cellules (RPMI+10%SVF) |
| C | 5µl d'IL2 à ... U/ml dans du milieu de culture |
| D | 5µl de cellules dans leur milieu de culture (concentration ... cellules/ml) |
| E | 5µl de cellules dans leur milieu de culture (concentration... cellules/ml) (duplicata D) |
| F | Idem D + 1 µl d'IL2 à ... U/ml |
| G | Duplicata F |
| H | |

Les échantillons (B à G) sont déposés sur la puce suivant le protocole précédent.

### Exemple 3: imagerie d'une partie du protéome de cellules (fibroblastes 3T3) après irradiation aux UV

Objectifs : de manière à connaître très rapidement les effets d'une drogue sur la réponse de cellules de la peau à des irradiations UV, ainsi que pour aider au ciblage de ces drogues sur un compartiment cellulaires particulier, il est intéressant d'étudier la répartition d'une partie des protéines exprimées par ces cellules.

Mise en oeuvre :
Des cellules (fibroblastes 3T3) sont cultivées en goutte sur une cible MALDI en acier inox (par exemple disponible chez Brüker Daltonics Inc.). Des gouttes de milieux de culture sans cellules sont déposées de la même façon sur la puce comme témoins. La puce est disposée dans une enceinte à atmosphère et température contrôlée (37°C, 100% H₂O, 5%CO₂).

On fabrique ainsi plusieurs puces.

On incube la puce pendant 24h de manière à laisser adhérer les cellules

Les gouttes sont ensuite irradiées de façon sélective au travers d'un masque adapté, ne laissant passer les UV que sur les plots supposés en recevoir, avec plusieurs temps d'irradiation (en changeant les masques au cours de l'expérience) et/ou plusieurs intensités (masque plus ou moins opaque).

Après la stimulation, on laisse les cellules en culture pendant plusieurs durées d'incubation (une puce par durée d'incubation) par exemple (0, 1h, 4h, 24h, et 48h) pour analyser l'évolution du phénotype.

Les échantillons sont alors congelés, additionnés de matrice (acide sinapinique par exemple) et séchés, puis introduits dans le spectromètre de masse pour y être analysés.

On récupère une moyenne de 50 spectres (équivalents à 50 tirs laser) par point (ou pixel), un point ayant une résolution de la taille du faisceau laser (inférieur à 25µm de diamètre). Pour chaque point le spectre nous donne un ensemble de pics. Un logiciel adéquat permet de sélectionner un pic d'intérêt, et on retranscrit une image de la répartition du composé décrit par ce pic sur l'ensemble de l'échantillon.

La masse des composés d'intérêt nous permet de remonter à sa nature (à l'aide de la consultation de bases de données), ou bien s'il s'agit d'un composé inconnu, on peut réitérer l'expérience en l'agrémentant d'analyse plus complètes (par exemple en faisant suivre les premiers spectres d'une analyse par MS multidimensionnelle).

### Exemple 4 : Imagerie de la répartition des ions calcium au sein de culture cellulaires par TOF-SIMS et laser SNMS

Intérêt : les ions calcium tendent à s'accumuler dans des cellules endommagées.

Mise en oeuvre :
Les cellules sont cultivées sur puce de silicium, soumises ou non à un ou plusieurs stress mécaniques, puis les échantillons sont cryogénisés.

Ils sont ensuite introduits dans le spectromètre sous vide (faisceau d'ions de 50 à 200 nm de diamètre) et balayés par le faisceau dans les mêmes conditions qu'à l'exemple 3.

L'exploitation des résultats suit les mêmes étapes que dans l'exemple précédent.

### Exemple 5: Mise en évidence d'un profil spectral correspondant à un phénotype particulier

Le but de cette expérience est d'illustrer la discrimination entre plusieurs phénotypes par spectrométrie de masse sur des gouttes de culture cellulaire. Nous avons étudié des phénomènes de cytotoxicité avec l'utilisation de CDDP (cisplatine = CIS-DIAMINEDICHLOROPLATINUM, propriété de l'unité INSERM 318), qui est un anti-cancéreux utilisé en chimiothérapie ; ainsi que l'apoptose (mort cellulaire programmée) avec l'utilisation du TNF (tumor necrosis factor)

Les puces utilisées font partie du système ProteinChip^{®} commercialisé par la société Ciphergen Inc. (voir exemple 2), utilisant la plateforme SELDI. Il s'agit plus particulièrement de la puce NP20 (référence commerciale : C553-0043 NP20 ProteinChip Array, A-H Format), dont la surface hydrophile est en silice.

Nous utilisons la lignée cellulaire U373 (disponible auprès du fournisseur ecace sous le numéro 89081403).

L'ensemble des étapes suivantes ont été conduites dans des conditions stériles.

Dans un premier temps, les puces NP20 ont été plongées dans l'alcool 70% pendant 20 min et séchées à la température ambiante sous hotte. Les cultures cellulaires (dans du milieu de culture DMEM + 10% de sérum de veau foetal) ont été déposées en gouttes de 5µl sur 2 puces comme indiqué dans le tableau ci-dessous.

| Spot | cellules | Traitements cytotoxicité (puce n°1) | Traitements apoptose (puce n°2) |
|---|---|---|---|
| A | Milieu de culture sans cellules | Avec CDDP (1µl : concentration finale 5.10⁻⁶) | Avec TNF |
| B | 20 000 cellules | | |
| C | 20 000 cellules | | |
| D | 20 000 cellules | | |
| E | Milieu de culture sans cellules | Sans CDDP | Sans TNF |
| F | 20 000 cellules | | |
| G | 20 000 cellules | | |
| H | 20 000 cellules | | |

On a ensuite laissé incuber les puces dans une étuve dans une atmosphère saturée en eau à 37°C sous 5% de CO₂ pendant un jour.
Puis conformément aux conditions exposées dans le tableau, on a déposé 1µl de CDDP dilué dans du milieu de culture cellulaire dans les gouttes A, B, C et D de la puce n°1, pour arriver à une concentration finale d'environ 5.10⁻⁶M. Sur la puce n°2 on a déposé 0.2µl de TNF dans les gouttes A, B, C et D pour obtenir une concentration finale d'environ 0.01µg/ml.
Les dispositifs ont alors été laissés à incuber comme précédemment à l'étuve pendant 1 jour.
Les manipulations suivantes ont été faites dans des conditions non stériles.
Avant l'introduction des puces dans le spectromètre de masse, elles ont été trempées dans une solution d'Hepes 2 mM. On a attendu qu'elles sèchent à la température ambiante (10-15 min).
On a préalablement préparé une solution de matrice (sinapinic acid) selon les protocoles recommandés par Ciphergen Inc. : la matrice a été dissoute dans 75µl d'acétonitrile et 75µl d'une solution aqueuse d'acide trifluoroacétique à 1%. La solution fut ensuite mélangée pendant 15min sur un vortex, puis centrifugée (10 000 tr/min) pendant 2 min juste avant son utilisation.
On a ajouté 2 fois 0.8 µl de matrice sur chaque plot, en laissant sécher entre chaque dépôt (3-5 min).
Lecture des puces :
Les puces ont alors été analysées dans le spectromètre de masse SELDI-TOF de Ciphergen.
On a voulu analyser toute la gamme de masse accessible au spectromètre. On a donc effectué trois analyses sur chaque puces pour 3 gammes de masses (low, medium, et high). Les spectres sont réalisés à l'aide du logiciel ProteinChip^{®} Software de Ciphergen Inc.
Les réglages sont effectués comme expliqué dans le tableau ci-dessous :

| passage | faible | moyen | haut |
|---|---|---|---|
| Intensité Laser | 178 | 198 | 230 |
| Hautes masses (en Da) | 100 000 | 200 000 | 300 000 |
| Déflecteur (en Da) | 1 500 | 3 000 | 5 000 |
| Sensibilité | 10 | 10 | 10 |
| Optimisation faibles masses (en Da) | 2 000 | 5 000 | 20 000 |
| Optimisation hautes masses (en Da) | 20000 | 50 000 | 150000 |

On a tiré sur les positions 21 à 81 pour les passages « faibles », par pas de 5, avec 2 coups d'échauffement (intensité+5) par position. Ils n'ont pas été inclus dans la moyenne des 15 tirs par positions qui, ajoutés, forment les spectres finaux. Les passages moyens utilisent les positions 22 à 82 et les passages « hauts », les positions 23 à 83.

Les résultats de ces essais sont illustrés par les figures 11,12 et 13.

**Figure 11** **:** exemple d'un spectre obtenu sans CDDP, sans TNF. En abscisse le rapport masse sur charge en Dalton (Da), en ordonnées l'intensité du signal (100 correspond à la saturation du détecteur).

**Figure 12** **:** représentation des différences entre les spectres des deux phénotypes sans TNF et avec TNF

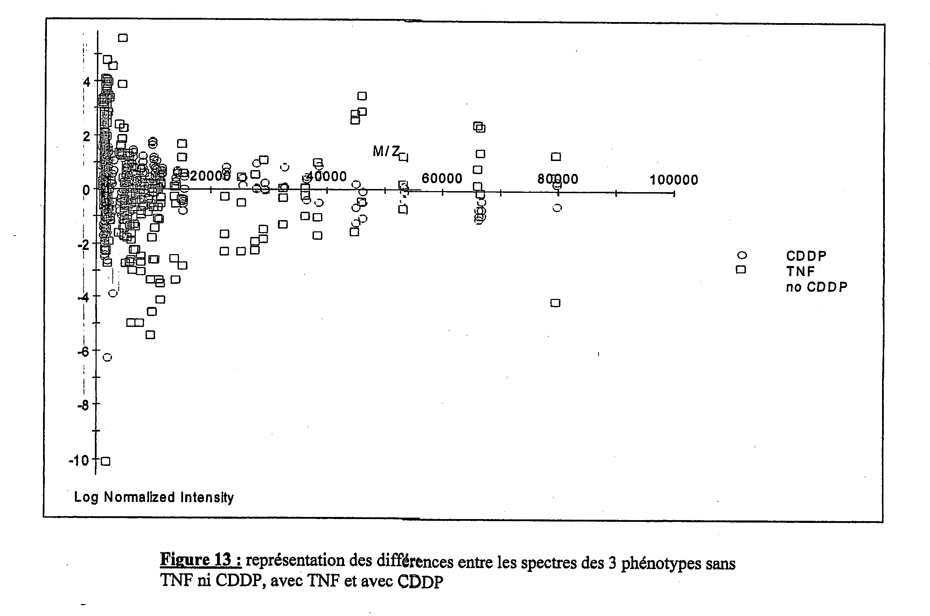

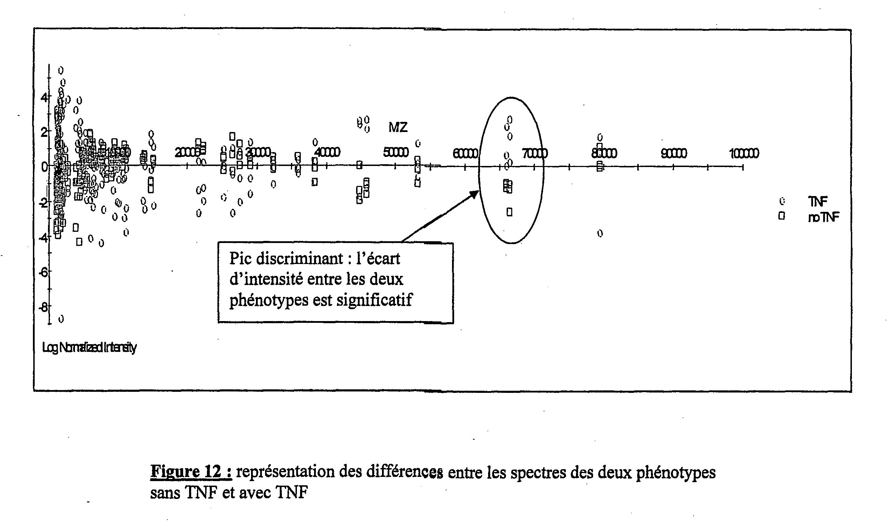

La figure 12 montre les différences d'intensité entre deux phénotypes pour chaque pic des spectres sur une échelle logarithmique : en bleu le profil de cellules stimulées par le TNF (donc en apoptose), en rouge le profil de cellules non stimulées. La figure 13 montre de la même manière en bleu le profil des cellules stimulées au TNF, en rouge celui des cellules stimulées au CDDP, et en vert celui des cellules non stimulées.

**Figure 13****:** représentation des différences entre les spectres des 3 phénotypes sans TNF ni CDDP, avec TNF et avec CDDP

Ces résultats montrent qu'il est possible de différentier non seulement des cellules non stimulées de cellules stimulées, mais aussi de différentier entre 2 phénotypes différents de manière significative.

De plus, de manière classique ces phénotypes n'auraient pas pu être étudiés avant plusieurs jours d'incubation. Les marquages se faisant au bout de 3 jours pour la cytotoxicité et de 6 jours pour l'apoptose.

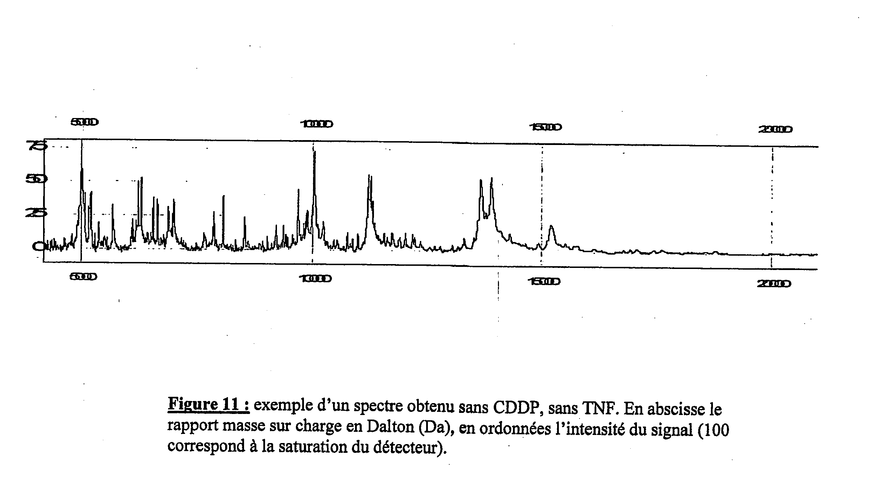

Les spectres obtenus (voir pour exemple la figure 11) ont ensuite été analysés pour vérifier que des signatures caractéristiques des phénotypes ont été obtenues.

## Revendications

1. Procédé d'analyse d'au moins un milieu réactionnel comportant au moins une cellule C, ledit procédé étant **caractérisé en ce que** :
On utilise un support S comportant une surface sensiblement plane, cette surface comportant au moins un moyen destiné à la réception des gouttes aqueuses,
(i) la cellule C est déposée sous forme d'une goutte aqueuse sur un moyen destiné à la réception des gouttes du support S, à l'aide d'un moyen choisi parmi : de fins capillaires, un système piezo électrique ;
(ii) la surface sensiblement plane du support S sur laquelle a été déposée la goutte aqueuse contenant la cellule C est recouverte par un film de séparation F, permettant le passage des gaz et empêchant l'évaporation des gouttes aqueuses déposées sur le support S ;
(iv) le milieu réactionnel est préparé et introduit dans le spectromètre de masse par l'introduction du support S dans le spectromètre de masse ;
(v) le milieu réactionnel est désorbé et ionisé ;
(vi) le spectre de masse du milieu réactionnel est recueilli et analysé.

2. Procédé selon la revendication 1, **caractérisé en ce que** dans une troisième étape (iii) la cellule C est soumise à une stimulation .

3. Procédé selon la revendication 2, **caractérisé en ce que** la stimulation à laquelle est soumise la cellule C est choisie parmi :
- l'introduction d'un réactif R,
- la mise en contact avec une ou plusieurs cellules,
- un apport d'énergie,
- l'application d'un champ électrique ou d'un champ magnétique,
- un traitement optique.

4. Procédé selon l'une quelconque des revendications 1 à 3, **caractérisé en ce que** l'accrochage des gouttes sur le support S se fait grâce aux forces de tension de surface.

5. Procédé selon l'une quelconque des revendications 1 à 4, **caractérisé en ce que** le dépôt des gouttes aqueuses contenant un réactif sur le support S, et éventuellement sous le film de séparation, se fait au moyen de fins capillaires.

6. Procédé selon l'une quelconque des revendications 1 à 4, **caractérisé en ce que** le dépôt des gouttes aqueuses contenant un réactif sur le support S se fait au moyen d'un système piézoélectrique.

7. Procédé selon l'une quelconque des revendications 3 à 6, **caractérisé en ce que** le réactif R est choisi parmi :
des molécules inorganiques, des molécules organiques naturelles, des molécules issues de synthèse organique et de synthèse combinatoire, des molécules extraites d'échantillons biologiques et des molécules extraites d'échantillons biologiques modifiées par synthèse, notamment les ADN simple et double brin, les ARN simple et double brin, les protéines et les peptides.

8. Procédé selon l'une quelconque des revendications 1 à 7, **caractérisé en ce qu'**il comporte une ou plusieurs étapes de traitement du milieu réactionnel directement sur le support S avant son introduction dans le spectromètre de masse.

9. Procédé selon la revendication 8, **caractérisé en ce qu'**il comporte au moins une étape de traitement choisie parmi : une lyse des cellules, un ou plusieurs lavages, l'adsorption ou la fixation de molécules.

10. Procédé selon l'une quelconque des revendications 1 à 9, **caractérisé en ce qu'**il comporte au moins une étape de traitement du ou des milieux réactionnels placés sur le support S par une solution de molécules promotrices de désorption.

11. Procédé selon l'une quelconque des revendications 1 à 10, **caractérisé en ce que** la préparation en vue de l'introduction dans le spectromètre de masse comporte au moins une étape sélectionnée parmi : une congélation du milieu réactionnel ; un séchage avec ou sans traitement à la chaleur et avec ou sans vide ; une fixation par un traitement à l'aide d'un agent tel que le méthanol ou le formaldéhyde.

12. Procédé selon l'une quelconque des revendications 1 à 11, **caractérisé en ce que** la préparation en vue de l'introduction dans le spectromètre de masse comporte l'addition au milieu réactionnel d'une ou de plusieurs molécules acides, de petite taille et absorbant la lumière suivie d'un séchage.

13. Procédé selon l'une quelconque des revendications 1 à 12, **caractérisé en ce que** qu'il comporte au moins les étapes suivantes :
- introduction du ou des milieux réactionnels placés sur le support S dans un tube d'introduction du spectromètre de masse ;
- application d'un vide et d'un champ électrique dans le tube de spectromètre;
- application d'un traitement de désorption/ionisation de façon contrôlée et séquencée sur le ou les échantillons ;
- détection de la masse des ions formés.

14. Procédé selon l'une quelconque des revendications 1 à 13, **caractérisé en ce qu'**il comporte au moins une étape de comparaison des données recueillies avec une banque de spectres de masse.

15. Dispositif permettant l'analyse d'au moins un milieu réactionnel comportant au moins une cellule C, ce dispositif étant **caractérisé en ce qu'**il comporte :
- un support S comportant une surface sensiblement plane, dont la surface est recouverte d'un film de séparation F permettant le passage des gaz et empêchant l'évaporation des gouttes aqueuses déposées sur le support S, cette surface sensiblement plane comportant au moins un moyen destiné à la réception des gouttes aqueuses, ce support S étant susceptible d'être introduit dans le spectromètre de masse,
- des moyens permettant le dépôt sur ladite surface, de gouttes aqueuses contenant la cellule C choisis parmi de fins capillaires ou un système piezo électrique,
- des moyens permettant la désorption et l'ionisation du milieu réactionnel,
- un spectromètre de masse.

16. Dispositif selon la revendication 15, **caractérisé en ce qu'**il comporte en outre une enceinte à atmosphère contrôlée dans laquelle est placé le support S de façon à permettre la survie de la cellule C.

17. Dispositif selon la revendication 16, **caractérisé en ce que** l'enceinte à atmosphère contrôlée est une étuve à température allant de 35 à 42°C, préférentiellement de 36,5 et 37,5°C, le taux de CO₂ est préférentiellement maintenu entre 3 et 5%, le taux d'oxygène O₂ est préférentiellement celui de l'air ambiant.

18. Dispositif selon la revendication 15, **caractérisé en ce que** le film F est choisi parmi :
- un liquide non miscible avec l'eau ;
- un gaz ;
- un film souple, solide ;
- un capot rigide alvéolé en matériau poreux, la taille des alvéoles étant adaptée pour pouvoir contenir la goutte de cellule(s) et éventuellement une goutte de réactif.

19. Dispositif selon l'une quelconque des revendications 15 à 18, **caractérisé en ce que** le support S est constitué par une plaque qui peut être en silicium, en verre ou en polymère.

20. Dispositif selon l'une quelconque des revendications 15 à 19, **caractérisé en ce que** le support comporte une couche électriquement conductrice.

21. Dispositif selon la revendication 20, **caractérisé en ce que** le moyen destiné à la réception des gouttes aqueuses consiste en l'un des moyens suivants :
- le support S présente sur sa surface plane un caractère hydrophobe et comporte une ou plusieurs zones hydrophiles ;
- le support S comporte sur sa surface plane des cavités, d'une profondeur allant de 1 micron à 1 millimètre ;
- le support S est une plaque munie d'excroissances de faible épaisseur, de 1 micron à 1 millimètre, disposées sur sa surface et destinées à favoriser l'accrochage des gouttes ;
- le support S est une plaque munie d'au moins un fil, sur lequel viennent s'accrocher les gouttes.

22. Dispositif selon l'une quelconque des revendications 15 à 21, **caractérisé en ce que** le support S du dispositif est mobile.

23. Dispositif selon l'une quelconque des revendications 15 à 22, **caractérisé en ce que** les gouttes aqueuses contenant une ou plusieurs cellules comportent un milieu de culture.

24. Dispositif selon l'une quelconque des revendications 15 à 23, **caractérisé en ce que** les moyens sont reliés à un dispositif de contrôle permettant son automatisation.

25. Dispositif selon l'une quelconque des revendications 15 à 24, **caractérisé en ce que** le support S comprend des moyens de réception des gouttes disposés de façon régulière sous forme de matrice.

26. Dispositif selon l'une quelconque des revendications 15 à 25, **caractérisé en ce qu'**il comporte au moins un appareil permettant de mesurer la masse d'un échantillon au moyen de la spectrométrie de masse, ledit appareil comprenant un tube de spectromètre, un dispositif permettant de faire le vide dans ledit tube, des moyens électriques permettant d'appliquer un potentiel d'accélération électrique dans le tube de façon à accélérer les molécules de l'échantillon à analyser, un moyen permettant de détecter la masse des ions formés, un moyen d'introduction du support S dans le tube, un moyen permettant la désorption et l'ionisation de l'échantillon à traiter.

27. Dispositif selon l'une quelconque des revendications 15 à 26, **caractérisé en ce que** le moyen de désorption est sélectionné parmi : un faisceau laser, un faisceau d'ions, un faisceau d'atomes neutres, un faisceau d'électrons, la nébulisation d'un échantillon liquide.

28. Dispositif selon l'une quelconque des revendications 15 à 27, **caractérisé en ce que** le moyen de désorption/ionisation est sélectionné parmi :
o MALDI : désorption et ionisation au laser assisté par la présence d'une matrice
o SELDI désorption et ionisation améliorées par une surface active
o SIMS spectrométrie de masse à ions secondaires
o SNMS spectrométrie de masse à neutres secondaires
o ESI ionisation par nébulisation
o FAB bombardement d'atomes rapides
o APCI ionisation chimique à pression atmosphérique

29. Dispositif selon l'une quelconque des revendications 15 à 28, **caractérisé en ce que** le moyen de mesure de la masse est sélectionné parmi :
o TOF : temps de vol
o MS/MS: spectrométrie de masse en tandem, spectrométrie de masse multidimensionnelle.
o Quadrupole : quadrupole ou trappe à ion
o FT-MS ou FT-ICR: spectrométrie de masse à transformée de Fourrier - caractérisation par résonance cyclotronique

30. Utilisation d'un dispositif selon l'une quelconque des revendications 15 à 29 pour identifier des modifications intervenues dans une culture cellulaire à la suite d'une stimulation.

31. Utilisation d'un dispositif selon l'une quelconque des revendications 15 à 29 pour étudier l'évolution dans le temps de la réponse d'une cellule ou d'un ensemble de cellules à une stimulation.

## Claims

1. Method for analysing at least one reaction medium comprising at least one cell C, said method being **characterised in that**:
there is used a support S comprising a substantially planar surface, that surface comprising at least one means for receiving aqueous drops,
(i) the cell C is deposited in the form of an aqueous drop on a drop-receiving means of the support S, with the aid of a means selected from: fine capillaries, a piezoelectric system;
(ii) the substantially planar surface of the support S on which the aqueous drop containing the cell C has been deposited is covered with a separating film F which allows gases to pass through and prevents the aqueous drops deposited on the support S from evaporating;
(iv) the reaction medium is prepared and introduced into the mass spectrometer by introducing the support S into the mass spectrometer;
(v) the reaction medium is desorbed and ionised;
(vi) the mass spectrum of the reaction medium is recorded and analysed.

2. Method according to claim 1, **characterised in that** in a third step (iii) the cell C is subjected to a stimulation.

3. Method according to claim 2, **characterised in that** the stimulation to which the cell C is subjected is selected from:
- the introduction of a reagent R,
- contact with one or more cells,
- a supply of energy,
- the application of an electric field or of a magnetic field,
- an optical treatment.

4. Method according to any one of claims 1 to 3, **characterised in that** the drops are attached to the support S by means of surface tension forces.

5. Method according to any one of claims 1 to 4, **characterised in that** the aqueous drops containing a reagent are deposited on the support S, and where appropriate beneath the separating film, by means of fine capillaries.

6. Method according to any one of claims 1 to 4, **characterised in that** the aqueous drops containing a reagent are deposited on the support S by means of a piezoelectric system.

7. Method according to any one of claims 3 to 6, **characterised in that** the reagent R is selected from:
inorganic molecules, natural organic molecules, molecules obtained by organic synthesis and by combinatorial synthesis, molecules extracted from biological samples and
molecules extracted from biological samples modified by synthesis, especially single- and double-stranded DNAs, single- and double-stranded RNAs, proteins and peptides.

8. Method according to any one of claims 1 to 7, **characterised in that** it comprises one or more steps of treating the reaction medium directly on the support S before it is introduced into the mass spectrometer.

9. Method according to claim 8, **characterised in that** it comprises at least one treatment step selected from: lysis of the cells, one or more washing steps, adsorption or fixing of molecules.

10. Method according to any one of claims 1 to 9, **characterised in that** it comprises at least one step of treating the reaction medium or media placed on the support S with a solution of molecules that promote desorption.

11. Method according to any one of claims 1 to 10, **characterised in that** the preparation with a view to introduction into the mass spectrometer comprises at least one step selected from: freezing of the reaction medium; drying with or without heat treatment and with or without a vacuum; fixing by treatment with an agent such as methanol or formaldehyde.

12. Method according to any one of claims 1 to 11, **characterised in that** the preparation with a view to introduction into the mass spectrometer comprises the addition to the reaction medium of one or more acid molecules which are small in size and absorb light, followed by drying.

13. Method according to any one of claims 1 to 12, **characterised in that** it comprises at least the following steps:
- introduction of the reaction medium or media placed on the support S into a mass spectrometer introduction tube;
- application of a vacuum and of an electric field in the spectrometer tube;
- application of a desorption/ionisation treatment to the sample or samples in a controlled and sequenced manner;
- detection of the mass of the ions formed.

14. Method according to any one of claims 1 to 13, **characterised in that** it comprises at least one step of comparing the recorded data with a bank of mass spectra.

15. Device permitting the analysis of at least one reaction medium comprising at least one cell C, the device being **characterised in that** it comprises:
- a support S comprising a substantially planar surface; the surface of which is covered with a separating film F which allows gases to pass through and prevents the aqueous drops deposited on the support S from evaporating, the substantially planar surface comprising at least one means for receiving aqueous drops, the support S being capable of being introduced into the mass spectrometer,
- means for depositing on said surface aqueous drops containing the cell C, selected from fine capillaries and a piezoelectric system,
- means permitting the desorption and ionisation of the reaction medium,
- a mass spectrometer.

16. Device according to claim 15, **characterised in that** it further comprises a controlled-atmosphere chamber in which the support S is placed in order to permit the survival of the cell C.

17. Device according to claim 16, **characterised in that** the controlled-atmosphere chamber is an incubator at a temperature ranging from 35 to 42°C, preferably from 36.5 to 37.5°C, the CO₂ level is preferably maintained between 3 and 5%, the oxygen O₂ level is preferably that of the ambient air.

18. Device according to claim 15, **characterised in that** the film F is selected from:
- a water-immiscible liquid;
- a gas;
- a flexible, solid film;
- a rigid alveolar cover made of porous material, the size of the cavities being adapted to be able to contain the drop of cell(s) and optionally a drop of reagent.

19. Device according to any one of claims 15 to 18, **characterised in that** the support S is constituted by a plate which can be made of silicon, of glass or of polymer.

20. Device according to any one of claims 15 to 19, **characterised in that** the support comprises an electrically conductive layer.

21. Device according to claim 20, **characterised in that** the means for receiving the aqueous drops comprises one of the following means:
- the support S has a hydrophobic nature on its planar surface and comprises one or more hydrophilic zones;
- the support S comprises on its planar surface cavities of a depth ranging from 1 micron to 1 millimetre;
- the support S is a plate equipped with protuberances of small thickness, from 1 micron to 1 millimetre, which are disposed on its surface and are to promote the attachment of the drops;
- the support S is a plate equipped with at least one wire to which the drops become attached.

22. Device according to any one of claims 15 to 21, **characterised in that** the support S of the device is movable.

23. Device according to any one of claims 15 to 22, **characterised in that** the aqueous drops containing one or more cells comprise a culture medium.

24. Device according to any one of claims 15 to 23, **characterised in that** the means are connected to a control device permitting its automation.

25. Device according to any one of claims 15 to 24, **characterised in that** the support S comprises means for receiving the drops, which means are disposed in a regular manner in the form of a matrix.

26. Device according to any one of claims 15 to 25, **characterised in that** it comprises at least one piece of equipment which allows the mass of a sample to be measured by means of mass spectrometry, said piece of equipment comprising a spectrometer tube, a device allowing a vacuum to be created in said tube, electrical means allowing an electrical acceleration potential to be applied in the tube in order to accelerate the molecules of the sample to be analysed, a means allowing the mass of the ions formed to be detected, a means for introducing the support S into the tube, a means permitting the desorption and ionisation of the sample to be treated.

27. Device according to any one of claims 15 to 26, **characterised in that** the desorption means is selected from: a laser beam, a beam of ions, a beam of neutral atoms, a beam of electrons, the nebulisation of a liquid sample.

28. Device according to any one of claims 15 to 27, **characterised in that** the desorption/ionisation means is selected from:
o MALDI: matrix-assisted laser desorption and ionisation
o SELDI: surface-enhanced laser desorption and ionisation
o SIMS: secondary ion mass spectrometry
o SNMS: secondary neutral mass spectrometry
o ESI: electrospray ionisation
o FAB: fast atom bombardment
o APCI: atmospheric pressure chemical ionisation.

29. Device according to any one of claims 15 to 28, **characterised in that** the means for measuring the mass is selected from:
o TOF: time of flight
o MS/MS: tandem mass spectrometry, multidimensional mass spectrometry
o Quadrupole: quadrupole or ion trap
o FT-MS or FT-ICR: Fourier transform mass spectrometry - characterisation by cyclotron resonance.

30. Use of a device according to any one of claims 15 to 29 for identifying modifications which have occurred in a cell culture following a stimulation.

31. Use of a device according to any one of claims 15 to 29 for studying the change over time in the response of a cell or of a group of cells to a stimulation.

## Patentansprüche

1. Verfahren zur Analyse von wenigstens einem Reaktionsmedium, das wenigstens eine Zelle C enthält, wobei das Verfahren **dadurch gekennzeichnet ist dass**:
ein Träger S verwendet wird, der eine im Wesentlichen ebene Fläche umfasst, wobei diese Oberfläche wenigstens ein Mittel zur Aufnahme von wässrigen Tropfen umfasst,
(i) die Zelle C in Form eines wässrigen Tropfens auf ein zur Aufnahme der Tropfen bestimmtes Mittel des Trägers S aufgetragen wird, und zwar mit Hilfe eines Mittels, das ausgewählt ist aus: feinen Kapillaren und einem piezoelektrischen System;
(ii) die im Wesentlichen ebene Fläche des Trägers S, auf die der wässrige, die Zelle C enthaltende Tropfen aufgetragen wurde, mit einem Trennfilm F bedeckt wird, der den Durchtritt von Gasen ermöglicht und das Verdampfen der wässrigen, auf den Träger S aufgetragenen Tropfen verhindert;
(iv) das Reaktionsmedium präpariert und durch Einführen des Trägers S in das Massenspektrometer in das Massenspektrometer eingeführt wird;
(v) das Reaktionsmedium desorbiert und ionisiert wird;
(vi) das Massenspektrum des Reaktionsmediums aufgenommen und analysiert wird.

2. Verfahren nach Anspruch 1, **dadurch gekennzeichnet, dass** die Zelle C in einem dritten Schritt (iii) einer Stimulation unterworfen wird.

3. Verfahren nach Anspruch 2, **dadurch gekennzeichnet, dass** die Stimulation, der die Zelle C unterworfen wird, ausgewählt ist aus:
- dem Einführen eines Reaktionsmittels R,
- dem Inkontaktbringen mit ein oder mehreren Zellen,
- einer Energiezufuhr,
- dem Anlegen eines elektrischen Feldes oder eines magnetischen Feldes,
- einer optischer Behandlung.

4. Verfahren nach einem der Ansprüche 1 bis 3, **dadurch gekennzeichnet, dass** die Haftung der Tropfen auf dem Träger S durch Oberflächenspannungskräfte erfolgt.

5. Verfahren nach einem der Ansprüche 1 bis 4, **dadurch gekennzeichnet, dass** das Auftragen der wässrigen, ein Reaktionsmittel enthaltenden Tropfen auf den Träger S und gegebenenfalls unter den Trennfilm F mittels feiner Kapillaren durchgeführt wird.

6. Verfahren nach einem der Ansprüche 1 bis 4, **dadurch gekennzeichnet, dass** das Auftragen der wässrigen, ein Reaktionsmittel enthaltenden Tropfen auf den Träger S mittels eines piezoelektrischen Systems durchgeführt wird.

7. Verfahren nach einem der Ansprüche 3 bis 6, **dadurch gekennzeichnet, dass** das Reaktionsmittel R ausgewählt ist aus:
anorganischen Molekülen, natürlichen organischen Molekülen, Molekülen aus organischer Synthese und kombinatorischer Synthese, Molekülen, die aus biologische Proben extrahiert wurden und Molekülen, die aus biologischen Proben extrahiert und durch Synthese modifiziert wurden, insbesondere einzelsträngiger und dopplesträngiger DNA, einzelsträngiger und doppelsträngiger RNA, Proteinen und Peptiden.

8. Verfahren nach einem der Ansprüche 1 bis 7, **dadurch gekennzeichnet, dass** es ein oder mehrere Behandlungsschritte des Reaktionsmediums direkt auf dem Träger S vor dessen Einführen in das Massenspektrometer umfasst.

9. Verfahren nach Anspruch 8, **dadurch gekennzeichnet, dass** es wenigstens einen Behandlungsschritt umfasst, der ausgewählt ist aus: Zelllyse, einem oder mehreren Waschschritten, Adsorption oder Fixierung von Molekülen.

10. Verfahren nach einem der Ansprüche 1 bis 9, **dadurch gekennzeichnet, dass** es wenigstens einen Behandlungsschritt des oder der auf den Träger S platzierten Reaktionsmedien mittels einer Lösung von Molekülen umfasst, die eine Desorption begünstigen.

11. Verfahren nach einem der Ansprüche 1 bis 10, **dadurch gekennzeichnet, dass** die Präparation im Hinblick auf das Einführen in das Massenspektrometer wenigstens einen Schritt umfasst, der ausgewählt ist aus: Einfrieren des Reaktionsmediums; _ Trocknen mit oder ohne Wärmebehandlung und mit oder ohne Vakuum; Fixierung durch Behandlung mit einem Mittel wie Methanol oder Formaldehyd.

12. Verfahren nach einem der Ansprüche 1 bis 11, **dadurch gekennzeichnet, dass** die Präparation im Hinblick auf das Einführen in das Massenspektrometer die Zugabe von ein oder mehreren sauren Molekülen, die klein sind und Licht absorbieren, zu dem Reaktionsmedium gefolgt von Trocknen umfasst.

13. Verfahren nach einem der Ansprüche 1 bis 12, **dadurch gekennzeichnet, dass** es wenigstens die folgenden Schritte umfasst:
- Einführen des oder der auf den Träger S platzierten Reaktionsmedien in eine Massenspektrometerröhre;
- Anlegen eines Vakuums und eines elektrischen Feldes in der Spektrometerröhre;
- kontrollierte und sequentielle Desorptions-/Ionisationsbehandlung der Probe(n);
- Detektion der Masse der gebildeten Ionen.

14. Verfahren nach einem der Ansprüche 1 bis 13, **dadurch gekennzeichnet, dass** es wenigstens einen Schritt zum Vergleich der aufgenommenen Daten mit einer Masseüspektrendatenbank umfasst.

15. Vorrichtung zur Analyse von wenigstens einem Reaktionsmedium, das wenigstens eine Zelle C enthält, wobei die Vorrichtung umfasst:
- einen Träger S, der eine im Wesentlichen ebene Fläche umfasst, deren Oberfläche mit einem Trennfilm F bedeckt ist, der den Durchtritt von Gasen ermöglicht und das Verdampfen von wässrigen, auf dem Träger S aufgetragenen Tropfen verhindert, wobei diese im Wesentlichen ebene Fläche wenigstens ein Mittel zur Aufnahme von wässrigen Tropfen umfasst, und der Träger S geeignet ist, in das Massenspektrömeter eingeführt zu werden,
- Mittel zum Auftragen von wässrigen, die Zelle C enthaltenden Tropfen auf die Oberfläche, ausgewählt aus feinen Kapillaren oder einem piezoelektrischen System,
- Mittel zur Desorption und Ionisation des Reaktionsmediums,
- ein Massenspektrometer.

16. Vorrichtung nach Anspruch 15, **dadurch gekennzeichnet, dass** sie außerdem eine Kammer mit kontrollierter Atmosphäre umfasst, in der der Träger S in einer Art und Weise platziert ist, die das Überleben der Zelle C ermöglicht.

17. Vorrichtung nach Anspruch 16, **dadurch gekennzeichnet, dass** die Kammer mit kontrollierter Atmosphäre ein Inkubator mit einer Temperatur von 35 bis 42°C, vorzugsweise 36,5 und 37,5°C, ist, wobei der CO₂-Gehalt vorzugweise zwischen 3 und 5% gehalten wird, und wobei der Sauerstoff O₂-Gehalt vorzugsweise der Gehalt der Umgebungsluft ist.

18. Vorrichtung nach Anspruch 15, **dadurch gekennzeichnet, dass** der Film F ausgewählt ist aus:
- einer mit Wasser nicht mischbaren Flüssigkeit;
- einem Gas;
- einem flexiblen, festen Film;
- einer starren zellenartigen Abdeckung aus porösem Material, wobei die Größe der Zellen so ist, dass sie den Tropfen mit der (den) Zelle(n) und gegebenenfalls einen Tropfen mit Reaktionsmitteln enthalten können.

19. Vorrichtung nach einem der Ansprüche 15 bis 18, **dadurch gekennzeichnet, dass** der Träger S aus einer Platte besteht, die aus Silizium, Glas oder Polymer sein kann.

20. Vorrichtung nach einem der Ansprüche 15 bis 18, **dadurch gekennzeichnet, dass** der Träger eine elektrisch leitfähige Schicht umfasst.

21. Vorrichtung nach Anspruch 20, **dadurch gekennzeichnet, dass** das zur Aufnahme der wässrigen Tropfen bestimmte Mittel aus einem der folgenden Mittel besteht:
- der Träger S zeigt auf seiner ebenen Oberfläche einen hydrophoben Charakter und umfasst einen oder mehrere hydrophile Bereiche;
- der Träger S umfasst auf seiner ebenen Oberfläche Vertiefungen mit einer Tiefe von 1 Mikron bis 1 Millimeter;
- der Träger S ist eine Platte, die mit Erhebungen geringer Höhe von einem Mikron bis 1 Millimeter versehen ist, die auf seiner Oberfläche angeordnet sind, um das Anhaften der Tropfen zu begünstigen;
- der Träger S ist eine Platte, die mit wenigstens einem Draht versehen ist, an dem die Tropfen haften.

22. Vorrichtung nach einem der Ansprüche 15 bis 21, **dadurch gekennzeichnet, dass** der Träger S der Vorrichtung beweglich ist.

23. Vorrichtung nach einem der Ansprüche 15 bis 22, **dadurch gekennzeichnet, dass** die wässrigen Tropfen, die ein oder mehrere Zellen enthalten, ein Kulturmedium umfassen.

24. Vorrichtung nach einem der Ansprüche 15 bis 23, **dadurch gekennzeichnet, dass** die Mittel mit einer Steuerungsvorrichtung verbunden sind, die ihre Automatisierung ermöglicht.

25. Vorrichtung nach einem der Ansprüche 15 bis 24, **dadurch gekennzeichnet, dass** der Träger S Mittel zur Aufnahme von Tropfen umfasst, die regelmäßig in Matrixform angeordnet sind.

26. Vorrichtung nach einem der Ansprüche 15 bis 25, **dadurch gekennzeichnet, dass** sie wenigstens ein Gerät zum Messen der Masse einer Probe mittels Massenspektrometrie umfasst, wobei das Gerät umfasst: eine Spektrometerröhre, eine Vorrichtung zur Erzeugung eines Vakuums in der Röhre, elektrische Mittel zum Anlegen eines elektrischen Potentials zur Beschleunigung in der Röhre, um die zu analysierenden Probenmoleküle zu beschleunigen, ein Mittel zur Detektion der Masse der gebildeten Ionen, ein Mittel zum Einführen des Trägers S in die Röhre, und ein Mittel zur Desorption und Ionisation der zu behandelnden Probe.

27. Vorrichtung nach einem der Ansprüche 15 bis 26, **dadurch gekennzeichnet, dass** das Mittel zur Desorption ausgewählt ist aus: einem Laserstrahl, einem lonenstrahl, einem Strahl neutraler Atome, einem Elektronenstrahl, und dem Versprühen einer flüssigen Probe.

28. Vorrichtung nach einem der Ansprüche 15 bis 27, **dadurch gekennzeichnet, dass** das Mittel zur Desorption/Ionisation ausgewählt ist aus:
- MALDI: Matrix-unterstützte Laser-Desorption/Ionisation
- SELDI: Oberfläche-erhöhte Laser-Desorption/Ionisation
- SIMS: Sekundärionen-Massenspektrometrie
- SNMS: Sekundärneutralteilchen-Massenspektrometrie
- ESI: Elektrospray-Ionisation
- FAB: Beschuss mit schnellen Atomen
- APCI: chemische Ionisation bei Atmosphärendruck

29. Vorrichtung nach einem der Ansprüche 15 bis 28, **dadurch gekennzeichnet, dass** das Mittel zum Messen der Masse ausgewählt ist aus:
- TOF: Flugzeit
- MS/MS: Tandem-Massenspektrometrie, mehrdimensionale Massenspektrometrie
- Quadrupol: Quadrupol oder Ionenfalle
- FT-MS oder FT-ICR: Fourier-Transform-Massenspektrometrie - Charakterisierung mittels Ionen-Cyclotron-Resonanz

30. Verwendung einer Vorrichtung nach einem der Ansprüche 15 bis 29 zur Identifikation von Modifikationen, die in einer Zellkultur als Folge einer Stimulation auftreten.

31. Verwendung einer Vorrichtung nach einem der Ansprüche 15 bis 29 zur Untersuchung der zeitlichen Änderung der Antwort einer Zelle oder einer Vielzahl von Zellen auf eine Stimulation.
